# EUROPEAN PATENT SPECIFICATION

(11) **EP 4 273 130 B1**
(45) Date of publication and mention of the grant of the patent: **03.12.2025**
(21) Application number: 21912357.7
(22) Date of filing: 17.12.2021
(51) Int. Cl.: C07D 233/64, B01D 53/62, B01D 53/14, C01B 32/50

(54) **PROCESS FOR SYNTHESIZING ZWITTERIONIC BASES, ZWITTERIONIC BASES, PROCESS FOR CAPTURING CO2 AND USE**
VERFAHREN ZUR SYNTHESE ZWITTERIONISCHER BASEN, ZWITTERIONISCHE BASEN, VERFAHREN ZUR ERFASSUNG VON CO2 UND VERWENDUNG
PROCÉDÉ DE SYNTHÈSE DE BASES ZWITTERIONIQUES, BASES ZWITTERIONIQUES, PROCÉDÉ DE CAPTURE DE CO2 ET UTILISATION

(30) Priority: 30.12.2020 BR 102020027071
(43) Date of publication of application: 08.11.2023
(73) Proprietor: Petróleo Brasileiro S.A. - Petrobras, 20031-912 Rio de Janeiro - RJ (BR); Universidade Federal Do Rio Grande Do Sul - UFRGS, Porto Alegre (BR)
(72) Inventor: FERREIRA DO NASCIMENTO, Jailton, 20031-912 Rio de Janeiro (BR); DUPONT, Jairton, 90040-060 Porto Alegre (BR); CABRAL DE MENEZES, Sonia Maria, 20031-912 Rio de Janeiro (BR); DOS SANTOS, Francisco Paulo, 90040-060 Porto Alegre (BR); MARIN, Graciane, 90040-060 Porto Alegre (BR); DE LEMOS PINTO AYDOS, Guilherme, 90040-060 Porto Alegre (BR); EBELING, Günter, 90040-060 Porto Alegre (BR)
(74) Representative: Clarke, Modet y Cía., S.L.
(86) International application number: PCT/BR2021/050557
(87) International publication number: WO 2022/140827

(56) References cited:
- KR-A- 20120 097 366
- US-A1- 2013 255 496
- EZZAT ABDELRAHMAN O ET AL: "Synthesis and application of new surface active poly (ionic liquids) based on 1,3-dialkylimidazolium as demulsifiers for heavy petroleum crude oil emulsions", JOURNAL OF MOLECULAR LIQUIDS, ELSEVIER, AMSTERDAM, NL, vol. 251, 17 December 2017 (2017-12-17), pages 201 - 211, XP085412014, ISSN: 0167-7322, DOI: 10.1016/J.MOLLIQ.2017.12.081
- SHAH SYED NASIR, LETHESH KALLIDANTHIYIL CHELLAPPAN, MUTALIB M. I. ABDUL, PILUS RASHIDAH BINTI MOHD: "Evaluation of Thermophysical Properties of Imidazolium-Based Phenolate Ionic Liquids", INDUSTRIAL & ENGINEERING CHEMISTRY RESEARCH, vol. 54, no. 14, 15 April 2015 (2015-04-15), pages 3697 - 3705, XP055955273, ISSN: 0888-5885, DOI: 10.1021/ie505059g
- LETHESH KALLIDANTHIYIL CHELLAPPAN, WILFRED CECILIA DEVI, TAHA M. F, THANABALAN M: "Synthesis and characterization of new class of ionic liquids containing phenolate anion", AIP CONFERENCE PROCEEDINGS, vol. 1621, 24 October 2014 (2014-10-24), NEW YORK, US , pages 261 - 266, XP055955276, ISSN: 0094-243X, DOI: 10.1063/1.4898476
- NASIR SHAH SYED, MUTALIB M. IBRAHIM. A., PILUS RASHIDAH BINTI MOHD, LETHESH KALLIDANTHIYIL CHELLAPPAN: "Extraction of Naphthenic Acid from Highly Acidic Oil Using Hydroxide-Based Ionic Liquids", ENERGY & FUELS, vol. 29, no. 1, 15 January 2015 (2015-01-15), pages 106 - 111, XP055955277, ISSN: 0887-0624, DOI: 10.1021/ef502169q
- CUI GUOKAI, WANG JIANJI, ZHANG SUOJIANG: "Active chemisorption sites in functionalized ionic liquids for carbon capture", CHEMICAL SOCIETY REVIEWS, vol. 45, no. 15, 1 January 2016 (2016-01-01), UK , pages 4307 - 4339, XP055955278, ISSN: 0306-0012, DOI: 10.1039/C5CS00462D
- WANG, C. ET AL.: "Tuning the basicity of ionic liquids for equimolar CO2 capture", ANGEWANDTE CHEMIE INTERNATIONAL EDITION, vol. 50, 2 March 2011 (2011-03-02), pages 4918 - 4922, XP055046683, DOI: 10.1002/anie.201008151

## Description

### Field of the Invention

The present invention addresses to cyclic sorption/desorption processes for the capture or purification of gaseous streams containing acidic gases using solutions of organic salts, herein called zwitterionic bases, with application in the energy, chemical and oil and gas fields in order to minimize the main intrinsic drawbacks in large-scale CO₂ and H₂S capture processes.

### Description of the State of the Art

The processes for capturing acidic gases, such as carbon dioxide (CO₂) and hydrogen sulfide (H₂S) are used in various industrial segments both for environmental reasons and for economic reasons, as they are used, for example, to obtain products with a higher added value or to purify the gaseous feed streams of catalytic reactors. These processes are especially important in the oil and gas industry, where they are used to remove CO₂ and H₂S from natural gaseous streams from oil extraction plants.

One of the widely disseminated technologies for the separation of CO₂ and H₂S from natural gas is based on the reversible absorption of these gases using aqueous solutions of ethanolamines such as, for example, methyldiethanolamine (MDEA). The use of amines, although effective, presents some intrinsic problems, the main ones being related to the volatility of these compounds, which causes mass loss of the absorbent agent during the process, the relative high enthalpy of absorption and their gradual thermal and chemical decomposition for the formation, for example, of carbamates. These drawbacks motivated the search for new compounds to replace amines both in new plants and in existing plants for capturing CO₂ and H₂S.

The main strategy studied to increase chemical and thermal stability, eliminate the mass loss by evaporation and reduce the enthalpy of absorption of the acidic gas capture processes has been the use of ionic compounds and their solutions. There is a growing number of publications reporting the use of ionic liquids (ILs), ionic liquid solutions or organic salt solutions in CO₂ absorption processes.

In one of the first studies that demonstrated the solubility of CO₂ in ionic liquids, BLANCHARD, L. A. et al. "Processing using ionic liquids and CO2", Nature, v. 399, p. 28-29, 1999 reported molar fractions of up to 0.72 moles of CO₂ for each mole of 1-butyl-3-methyl-imidazolium hexafluorophosphate (BMIm·PF6) under pressures of 83 bar (8.3 MPa). Since then, numerous publications have demonstrated that CO₂ is remarkably soluble in ILs based on imidazolium cations, as described in the references of ANTHONY, J. L.; MAGINN, E. J.; BRENNECKE, J. F. "Gas solubilities in 1-n-butyl-3-methylimidazolium hexafluorophosphate", Ionic Liquids, p. 260-269, 2002; Cadena, C., et al. "Why is CO2 so soluble in imidazolium-based ionic liquids?", Journal of the American Chemical Society, v. 126, p. 5300-5308, 2004; BARA, J. E. et al. "Roomtemperature ionic liquids and composite materials: platform technologies for CO2 capture", Accounts of Chemical Research, v. 43, p. 152-159, 2010; HASIB-UR-RAHMAN, M. et al. "Ionic liquids for CO2 capture - Development and progress", Chemical Engineering and Processing: Process Intensification, v. 49, p. 313-322, 2010; BRENNECKE, J. F.; GURKAN, B. E. "Ionic Liquids for CO2 Capture and Emission Reduction", The Journal of Physical Chemistry Letters, v. 1, p. 3459-3464, 2010; SHARMA, P. et al. "Effects of anions on absorption capacity of carbon dioxide in acid functionalized ionic liquids", Fuel Processing Technology, v. 100, p. 55-62, 2012; ZHANG, X. et al. "Carbon capture with ionic liquids: overview and progress", Energy & Environmental Science, v. 5, p. 6668-6681, 2012. The absorption of CO₂ in ionic liquids can occur either by physisorption or by chemisorption, or by a combination thereof. The physical absorption, or gas solubility, is influenced by a number of factors such as free volume, counterion size, strength of cation-anion interactions, pressure, temperature, and structural changes in the ionic pair. The chemisorption is normally influenced by the basicity of the salt and leads to the capture of CO₂ by the formation of species such as bicarbonate and carbamate. Therefore, when using ionic liquids or functionalized organic salts with basic groups, it is expected to combine the effects of physical and chemical absorption.

In a recent literature review, CUI, G. et al., "Active chemisorption sites in functionalized ionic liquids for carbon capture", Chemical Society Reviews, v. 45, p. 4307-4339, 2016 report the existence of at least 77 different amino-functionalized ionic liquids, 102 ionic liquids free of the amine group (including those that use the phenolate anion) and 55 ionic liquids with multiple active sites that were used to capture CO₂. Although these ionic liquids have interesting properties, so far there is no proof of the industrial viability of these compounds, due to several factors such as, for example, high viscosity, complexity of synthesis processes, incompatibility with water.

In the specific case of amino-functionalized ionic liquids, the advantage over alkanolamines is related only to the low volatility of these compounds. This advantage is not enough to compensate the disadvantages related to its high viscosity and cost, mainly because, as they have the same functional group, they do not represent gains in thermal and chemical stability.

Ionic liquids with alternative functional groups, specifically the phenolate anion, draw attention because they eliminate decomposition via the formation of carbamates; however, in the presence of water or another source of protons, the phenolate is converted to phenol, a volatile and toxic compound, making its industrial use unfeasible.

The alternative to the use of phenolate groups without the formation of volatile products is the use of inner salts. In the study by Sakai, T. et al. "Bifunctional organocatalyst for Activation of carbon dioxide and epoxy to produce cyclic carbonate: betaine as a new catalytic Motif", Organic Letters, v. 12, p. 5728-5731, inner salts containing the phenolate anion covalently associated with quaternary ammonium-type cations have been reported. Such a study is focused on the nucleophilicity of these compounds for the formation of adducts with CO₂ that are intermediates in coupling reactions of carbon dioxide with epoxides for the formation of cyclic carbonates, without investigating absorption of CO₂ under industrial conditions with such compounds.

A method for removing CO₂ from exhaust gases using ionic liquids associated with anions containing carboxylate groups was disclosed by patent US7527775B2. More recently, patent US8721770 describes the use of functionalized ionic liquids in CO₂ capture. Although promising, the use of ionic liquids for CO₂ absorption has the inconvenience of the high viscosity of these compounds, which, in practice, would make pumping and flow of these compounds difficult in industrial pipes and would impair mass transfer in the liquid.

An evolution of these methodologies is the use of solutions, including aqueous solutions, of ionic liquids and other organic salts that have lower viscosity than pure ionic liquids and similar or even greater capture capacity. One of the most important methods in the field of the present discovery was disclosed in the patent US8536371B2, which refers to the use of aqueous solutions of amino-functionalized organic salts for the capture of CO₂.

Furthermore, in 2017, SIMON, N. M. et al. "Carbon dioxide capture by aqueous ionic liquid solutions", ChemSusChem, v. 10, p. 4927-4933, 2017 reported, for example, the capture of CO₂ through aqueous solutions of ionic liquids containing imidazolium cations associated with acetate and imidazolate anions. Some other examples of CO₂ absorption methods by ionic compounds are reported in references US2005/0129598A1, US10086331B2, US2012/0063977A1, US2015/0314235A1, US2018/0179157A1 and WO2014/144523A3.

Document BR122012033196A2 refers to a method for absorbing CO₂ from a gaseous mixture, preferably a combustion exhaust gas containing from 1 to 60% by volume of CO₂, and also to the absorption medium and a device to perform the method, where absorption of CO₂ is carried out by contacting a gaseous mixture with an absorption medium comprising water and at least one amine. The method uses zwitterionic surfactants for CO₂ absorption, such as betaines, alkylglycines, sultaines, amphopropionates, amphoacetates, tertiary amine oxides and silicobetaines.

Patent US8741246B2 discloses methods for preparing compositions in order to capture volatile compounds in industrial power generation and commercial natural gas production facilities. More specifically, systems for the reduction of volatile compounds, where the system comprises an N-functionalized imidazole and may optionally include an amine. The method uses the non-ionic N-functionalized imidazole under neutral conditions and about 20% to 80% by weight of the solvent system. In addition, this document discloses a process of absorption of CO₂ and other volatile compounds, such as H₂S, with the regeneration of N-functionalized imidazole through heating. However, the use of ethanolamine compounds, although effective, presents some intrinsic problems, the main ones being related to the volatility of these compounds, which causes mass loss of the absorbent agent during the process, the relative high enthalpy of absorption and its gradual thermal and chemistry decomposition for the formation.

Document US9586175B2 discloses a process for separating at least a portion of an acidic gas from a gaseous mixture, comprising contacting the gaseous mixture with an absorption medium and/or adsorption medium, being carried out in the presence of an acidic catalyst. The described absorption and regeneration method uses zwitterionic substances providing compositions, devices and apparatus for capturing acidic gases, such as carbon dioxide (CO₂), from flue gas flows, reform gas flows, natural gas flows or other industrial gas flows.

The study by YEBEDRI, S. et al. "Characterizations of crystalline structure and catalytic activity of zwitterionic imidazole derivatives", Journal of Molecular Structure, v. 1193, p. 45-52, 2019 discloses the synthesis of a zwitterionic imidazole derivative and studies of catecholase activity by copper complexes *in situ.* Such a study focuses on the characterization of the crystalline structure and its catalytic activity, not being used on an industrial scale in the absorption of CO₂.

Korean patent application KR20120097366 discloses the use of imidazolium phenolates without covalent link between the imidazolium and phenolate for CO₂-capture.

Ezzat et al. (J. of Molecular Liquids, vol. 251, 2017, p. 201-211), a document not concerned with CO₂-capture, discloses the preparation of imidazolium phenols as intermediates in the preparation of poly(ionic liquids).

Thus, no document of the state of the art discloses a process for capturing CO₂ through solutions of zwitterionic bases such as that of the present invention.

In order to solve such problems, the present invention was developed, by obtaining a very specific family of organic salts, whose aqueous solutions have low viscosity and molar absorption capacity similar to conventional absorbents, such as MDEA, with the advantage of not being volatile, being less susceptible to chemical and thermal decomposition, as well as having a lower enthalpy of absorption. Such unique characteristics make them different from the aforementioned examples or from any other compound already used for the capture of acidic gases, especially carbon dioxide and hydrogen sulfide.

The present invention results in economic advantages when compared to traditional processes that use MDEA, such as, elimination of costs corresponding to the evaporation of the absorbent species, since the zwitterionic bases, as well as their conjugated acids, are not volatile; the reduction of about 20% of energy required for the desorption processes with zwitterionic solutions and the functional groups of the zwittwerions are extremely robust.

In addition, the invention has environmental and safety advantages that are related to the non-volatility of the inner salts, thus eliminating risks of possible human and environmental contaminations.

### Brief Description of the Invention

The present invention addresses to the discovery of a family of inner salts with a basic character, whose solutions are used in cyclic processes of gas capture on a large scale, through the contact of a gaseous stream containing one or more acidic gases with such solutions in order to minimize the main intrinsic inconveniences in large-scale CO₂ and H₂S capture processes.

These inner salts, or zwitterionic bases, can be combined with different solvents, including water, to obtain low viscosity solutions that have molar absorption capacity similar to conventional absorbents, such as MDEA, with the advantage of not being volatile, being less susceptible to chemical and thermal decomposition, in addition to having a lower enthalpy of absorption.

The present invention can be applied in several industrial segments, such as in the energy sector for capturing CO₂ from exhaust gases, in the chemical sector for removing CO₂ from gaseous streams of catalytic processes in which CO₂ can poison the catalysts and, especially, in the oil and gas sector for the purification of natural gas.

### Brief Description of the Drawings

The present invention will be described in more detail below, with reference to the attached figures which, in a schematic way and not limiting the inventive scope, represent examples of its embodiment. In the drawings there are:
- Figure 1 illustrating developed inner salts: A) 1,3-dimethyl-2-(3-oxy-phenyl)-imidazolium and B) 1,3-dimethyl-2-(4-oxy-phenyl)-imidazolium;
- Figure 2 illustrating the first step of the synthesis of the 1,3-dimethyl-2-(3-oxy-phenyl)-imidazolium (synthesis of 1-methyl-2(3-hydroxy-phenyl)-imidazole) inner salt;
- Figure 3 illustrating the second step of the synthesis of the 1,3-dimethyl-2-(3-oxy-phenyl)-imidazolium inner salt (synthesis of 1,3-dimethyl-2(3-hydroxy-phenyl)-imidazolium iodide);
- Figure 4 illustrating the ESI(+)MS spectrum of the 1,3-dimethyl-2(3-hydroxyphenyl)-imidazolium iodide precursor for the synthesis of the 1,3-dimethyl-2-(3-oxy-phenyl)-imidazolium inner salt, where there are represented the simulated spectrum at the top and below the experimentally obtained spectrum;
- Figure 5 illustrating the third step of the synthesis of the 1,3-dimethyl-2-(3-oxy-phenyl)-imidazolium inner salt (synthesis of the 1,3-dimethyl-2-(3-oxy-phenyl) - imidazolium inner salt);
- Figure 6 illustrating the NMR spectrum of ¹H NMR (400 MHz, D₂O) of 1,3-dimethyl-2-(3-oxy-phenyl)-imidazolium inner salt;
- Figure 7 illustrating the ¹³C NMR spectrum (100 MHz, D₂O) of 1,3-dimethyl-2-(3-oxy-phenyl)-imidazolium inner salt;
- Figure 8 illustrating the infrared spectrum of 1,3-dimethyl-2-(3-oxy-phenyl)-imidazolium inner salt;
- Figure 9 illustrating the first step of the synthesis of the 1,3-dimethyl-2-(4-oxy-phenyl)-imidazolium (synthesis of 1-methyl-2-(4-hydroxy-phenyl)-imidazole) inner salt;
- Figure 10 illustrating the second step of the synthesis of the 1,3-dimethyl-2-(4-oxy-phenyl)-imidazolium inner salt (synthesis of 1,3-dimethyl-2-(4-hydroxy-phenyl)-imidazolium iodide);
- Figure 11 illustrating the third step of the synthesis of the 1,3-dimethyl-2-(4-oxy-phenyl)-imidazolium inner salt (synthesis of the 1,3-dimethyl-2-(4-oxy-phenyl) - imidazolium inner salt);
- Figure 12 illustrating the NMR spectrum of ¹H NMR (400 MHz, DMSO-d₆) 1,3-dimethyl-2-(4-oxyphenyl)-imidazolium inner salt;
- Figure 13 illustrating the ¹³C NMR spectrum (100 MHz, DMSO-d₆) 1,3-dimethyl-2-(4-oxy-phenyl)-imidazolium inner salt;
- Figure 14 illustrating the infrared spectrum of 1,3-dimethyl-2-(4-oxy-phenyl)-imidazolium inner salt;
- Figure 15 illustrating the experimental apparatus for sorption tests, where there are represented the following items: 401 - CO₂ cylinder; 402 - CO₂ reservoir with monitored pressure; 403 - valve for pressure control in the sorption vessel; 404 - jacketed sorption vessel; 405 - purge valve; 406 - purge gas; 407 - thermostatic bath to control the temperature of the process; 408 - magnetic stirrer; 409 - Field Logger pressure recorder; and 410 - computer for data storage and processing;
- Figure 16 illustrating the UV-VIS spectra of the aqueous solution of the 1,3-dimethyl-2-(3-oxy-phenyl)-imidazolium inner salt, solution after absorption and after desorption;
- Figure 17 illustrating the recyclability of the aqueous solution of 1,3-dimethyl-2-(3-oxy-phenyl)-imidazolium, where the reaction conditions are: 5 mL of solution with a concentration of 1 M, absorption temperature of 40 °C and pressure of 1.3 bar absolute (130 kPa); desorption temperature of 100 °C and atmospheric pressure; absorption values determined through the pressure drop in the reservoir and expressed in moles CO₂ per moles of absorbent; mean represents mean absorption value from cycles 2 to 27 ± standard deviation.
- Figure 18 illustrating the recyclability of the aqueous solution of 1,3-dimethyl-2-(4-oxy-phenyl)-imidazolium, where the reaction conditions are: 5 mL of solution with a concentration of 1 M, absorption temperature of 40 °C and pressure of 1.3 bar absolute (130 kPa); desorption temperature of 100 °C and atmospheric pressure; absorption values determined through the pressure drop in the reservoir and expressed in moles CO₂ per moles of absorbent; mean represents mean absorption value from cycles 2 to 17 ± standard deviation;
- Figure 19 illustrating the recyclability of the commercial MDEA/Piperazine aqueous solution, where the reaction conditions are: 5 mL of MDEA/piperazine/water 40%:10%:50% solution, absorption temperature of 40 °C and pressure of 1.3 bar absolute (130 kPa); desorption temperature of 100 °C and atmospheric pressure; absorption values determined through the pressure drop in the reservoir and expressed in moles CO₂ per moles of absorbent; mean represents mean absorption value from cycles 1 to 26 ± standard deviation;

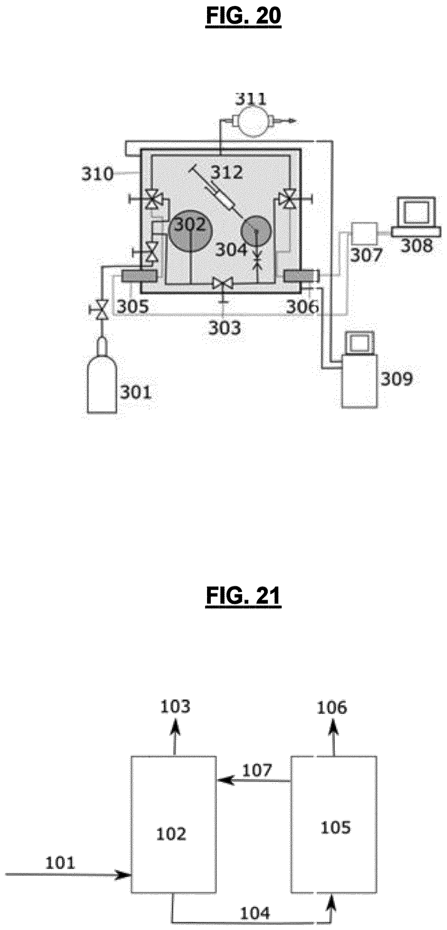

- Figure 20 illustrating the experimental apparatus for estimating the enthalpy of absorption, where there are represented the following items: 301 - CO₂ cylinder; 302 - CO₂ reservoir with monitored pressure; 303 - CO₂ admission valve in the sorption vessel; 304 - sorption vessel; 305 - pressure transducer to monitor the pressure in the CO₂ reservoir; 306 - pressure transducer to monitor the pressure of the sorption vessel; 307 - Field Logger pressure data recorder; 308 - computer for storing and processing data; 309 - thermostatic bath to control the temperature of the system; 310 - isochoric balance chamber; 311 - vacuum pump; and 312 - syringe for adding the sorbent solution;
- Figure 21 illustrating the process of absorption of acidic gases using zwitterionic bases, where there are represented the following items: 101 - gaseous feeding; 102 - absorption unit operating at T₁, P₁; 103 - purified gaseous stream; 104 - zwitterionic solution rich in absorbed acidic gases; 105 - desorption unit operating at T₂, P₂; 106 - gaseous stream rich in CO₂ and/or other acidic gases; and 107 - regenerated zwitterionic solution;
- Figure 22 illustrating the family of inner salts of the present invention with predictable effect for acidic gas capture processes from the mere direct extrapolation of the state of the art;
- Figure 23 illustrating the process of synthesis of zwitterionic bases of the present invention;
- Figure 24 illustrating the family of inner salts obtained by the process of synthesis of zwitterionic bases of the present invention;
- Figure 25 illustrating the ionic liquids used as a solvent of general formula A⁺Z⁻ for preparing the solutions of zwitterionic bases of the present invention;
- Figure 26 illustrating an embodiment of acidic gas absorption process using zwitterionic bases for the purification of natural gas, where there are represented the following items: 201 - gas feed; 202 - absorption unit at T₁, P₁; 203 - purified gaseous stream; 204 - zwitterionic solution rich in absorbed acidic gases; 205 - desorption unit operating at T₂, P₂; 206 - gaseous stream rich in CO₂ and/or other acidic gases; 207 - regenerated zwitterionic solution; 208 - reboiler; and 209 - heat exchanger.

### Detailed Description of the Invention

The process of synthesizing zwitterionic salts according to the present invention is characterized by comprising three very simple steps that occur successively promoting reactions between its components in the liquid phase, in the presence of solvent, under mechanical stirring, under pressure atmospheric and at temperatures between -10 °C and 100 °C, typically between 0 °C to 70 °C.

### a) First step:

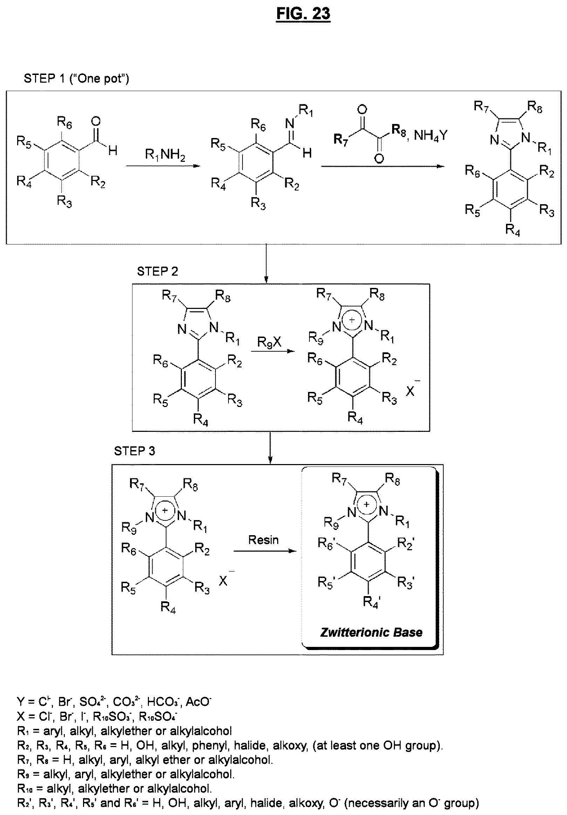

The first step is characterized by promoting the reaction between a compound of the hydroxy-benzaldehyde type with a primary amine of general formula R1-NH₂ for the formation of the corresponding imine, followed by the cyclization reaction of this imine, in the presence of a salt of ammonium and a vicinal dialdehyde, to obtain a compound of the 2-(hydroxy-phenyl)-imidazole type, according to Step 1 - Figure 23.

The first step is characterized by occurring at temperatures that can vary between -10 °C and 80 °C, typically 0 °C to 40 °C and reaction times between 10 minutes and 24 hours, typically 24 hours.

The first step is characterized by the reaction product of the 2-(hydroxy-phenyl)-imidazole type being purified by simple techniques of precipitation, crystallization and/or filtration followed, or not, by washing and/or evaporation of the solvent.

The compound of the hydroxy-benzaldehyde type is characterized by containing, in its aromatic ring, at least one hydroxyl group and the other substituents of the benzene ring being hydrogen, alkoxy groups containing between 1 and 10 carbons, halides, phenyl or alkyl group or containing between 1 and 10 carbons, with 3-hydroxy-benzaldehyde and 4-hydroxy-benzaldehyde being typically used.

The primary amine of general formula R1-NH₂ is characterized in that the radical R1 comprises an alkyl, aryl, alkylether or alkylalcohol group containing between 1 and 10 carbons, typically methylamine, and in that being used in the proportion of 0.5 to 2 equivalents to benzaldehyde, typically 1.05 equivalents.

The ammonium salt is characterized by having the general formula (NH₄)ₙY, where Y comprises the bromide, chloride, sulfate, acetate, carbonate and bicarbonate anions; n has a value of 1 or 2 and, because this salt is used in a proportion of 0.3 to 2 equivalents relative to the benzaldehyde, typically 0.75 equivalents.

Dialdehyde is characterized by being typically glyoxal and can also be analogues of glyoxal with alkyl, aryl, alkylether or alkylalcohol substituents containing 1 to 10 carbons and for being used in the proportion of 0.5 to 2 equivalents in relation to benzaldehyde, typically 1 equivalent.

The solvent used in the first step comprises water, acetonitrile, ethyl acetate, aliphatic alcohols containing from 1 to 10 carbons in the alkyl chain or a mixture of these in any proportion in the amount of 1 time to 100 times the mass of benzaldehyde, typically 10 times.

The purification of the reaction products of the first step is characterized by using water, acetonitrile, ethyl acetate, aliphatic alcohols containing from 1 to 10 carbons in the alkyl chain or a mixture of these in proportions suitable for, under temperatures of -10 °C to 30 °C, typically 0 °C, the precipitation or crystallization of compounds of the 2-(hydroxy-phenyl)-imidazole type is carried out followed by removal of the solvent by filtration or simple decantation, being recommended to wash the product with the same solvent and evaporating the residual solvent under reduced pressure.

### b) Second step:

The second step is characterized by promoting the N-alkylation reaction between the compound of the 2-(hydroxy-phenyl)-imidazole type obtained in the previous step and an alkylating agent of general formula R9-X to form a salt of 2-(hydroxy-phenyl)-imidazolium, in the presence of solvent, according to Step 2 - Figure 23.

The second step is characterized by occurring at temperatures that can vary between 40 °C and 100 °C, typically 70 °C, and reaction times between 10 minutes and 24 hours, typically 1 hour.

The second step is characterized in that the reaction product of the 2-(hydroxyphenyl)-imidazolium salt type precipitates as it is formed and is removed from the reaction mixture by filtration or decantation followed by washing and evaporation of the residual solvent under reduced pressure.

The alkylating agent of general formula R9-X is characterized in that R9 comprises an alkyl, arylalkyl, alkylether or alkylalcohol group containing between 1 and 10 carbons, and the X group comprises the halide, alkylsulfonate and alkylsulfate group, in which the alkyl group has between 1 and 5 carbons, typically iodomethane or methanesulfonyl chloride.

The solvent used in the second step comprises water, acetonitrile, ethyl acetate, aliphatic alcohols containing from 1 to 10 carbons in the alkyl chain or the mixture of these in appropriate proportions in the amount of 1 time to 20 times the mass of the compound of 2-(hydroxy-phenyl)-imidazole type, typically 5 times.

### c) Third step:

The third step is characterized by promoting the deprotonation of the phenolic OH group of the 2-(hydroxy-phenyl)-imidazolium salt, obtained in the previous step, through the reaction of an aqueous solution of this component with a strongly basic ion exchange resin, which results in the formation of the inner or zwitterionic salt of the 2-(oxy-phenyl)-imidazolium type, as shown in Step 3 - Figure 23.

The third step is also characterized in that the reaction between the 2-(hydroxyphenyl)-imidazolium salt and the ion exchange resin occurs by simply mixing and stirring these components for periods of up to 60 minutes, typically 10 minutes, or by passing a solution of the 2-(hydroxy-phenyl)-imidazolium salt through a fixed or fluidized bed column of resin at temperatures between 10 °C and 80 °C, typically 30 °C

Furthermore, the third step is characterized in that the aqueous solution containing the product is separated from the ionic resin by filtration or simple decantation and the reaction product, inner salt of the 2-(oxy-phenyl)-imidazolium type, is isolated by simple evaporation of the solvent under reduced pressure and temperatures between 10 °C and 80 °C, typically 40 °C.

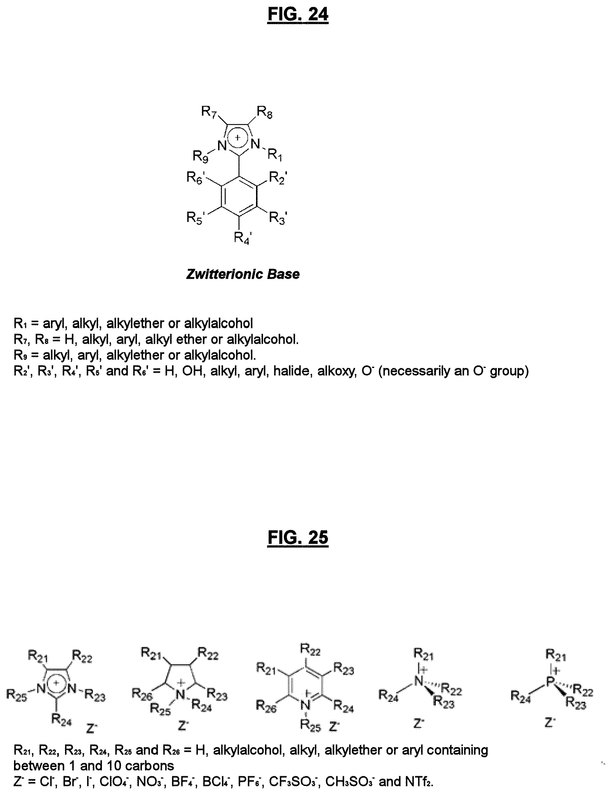

The zwitterionic bases obtained in the synthesis process disclosed herein have as their main structural characteristic the presence of an imidazolium cation covalently linked, through carbon C-2, to an oxy-phenyl group, having as possible substituents of the imidazolium ring, R₇ and R₈, hydrogen or alkyl, aryl, alkylether or alkylalcohol groups containing 1 to 10 carbons; and as substituents on the benzene ring, necessarily an O⁻group, the others being hydrogen, hydroxyl, alkoxy groups containing between 1 and 10 carbons, halide, phenyl or alkyl group containing between 1 and 10 carbons, as shown in Figure 24.

The zwitterionic bases obtained in the synthesis processes are neutral compounds that have opposite charges in different regions of their structure and can also be called inner salts or hybrid salts.

The zwitterionic bases obtained in the synthesis processes are solids soluble in water and polar organic solvents, such as alcohols, polyalcohols, acetonitrile and acetone.

Unlike most traditional zwitterions, which are amphoteric amino acids, the main property of zwitterionic bases is precisely the fact that they are basic inner salts, and have relatively high basicity, typical of the phenolate group, and may vary depending on the substituents of the benzene ring.

The zwitterionic bases have a unique characteristic for the process of capturing acidic gases, the fact that both they and their acid-conjugates are organic salts, therefore, non-volatile.

The key aspect of the zwitterionic bases disclosed herein is the presence of a phenolate group covalently linked to an imidazolium cation to form a basic salt with good solubility in water and polar solvents.

The process for capturing acidic gases through zwitterionic base solutions comprises contacting, under sorption conditions, a gaseous stream containing one or more acidic gases with a zwitterionic base sorbent solution for the removal of at least part of the acidic gases, and obtaining a purified gaseous stream and a solution with sorbed acidic gases; and the regeneration of the sorbent solution, under desorption conditions, to obtain a zwitterionic base solution and a stream of acidic gases.

Figure 21 illustrates in a simplified way the continuous cyclic process of purification of a gaseous stream using solutions of zwitterionic bases. The feed stream **101** enters the sorption unit **102,** which contains the zwitterionic base solution and whose temperature is controlled by heat extraction. In the sorption unit, the gaseous stream comes into contact with the zwitterionic base solution under temperature and pressure conditions (T1 and P1) so that at least part of the acidic gases contained in the feed stream is absorbed by the zwitterionic base solution, generating a purified gaseous stream **103** and a solution rich in sorbed acidic gases **104,** which is conducted to the desorption unit **105.** In the desorption unit, acidic gases are desorbed from the solution through appropriate temperature and pressure conditions (T2 and P2) generating a gaseous stream rich in acidic gases **106** and the regenerated zwitterionic base solution **107.**

Conventional equipment can be used for the various functions necessary for the process of capturing acidic gases by solutions of zwitterionic bases, such as, for example, to control the gaseous flow, to promote contact between the gas and the sorbent solutions, for pumping the solutions between the sorption and desorption units, to promote heat exchanges between the process streams in such a way as to obtain an efficient cyclic process of capturing acidic gases.

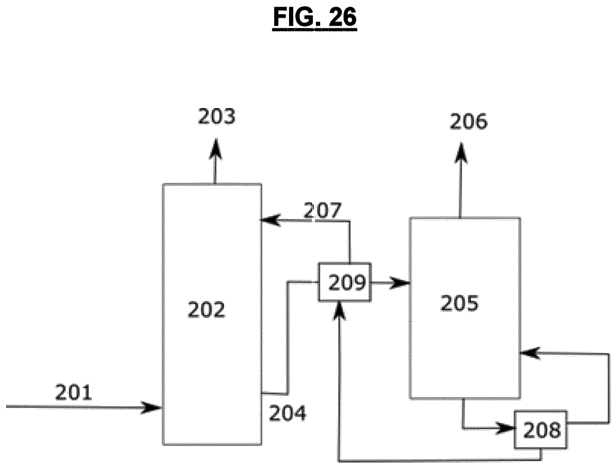

Figure 26 shows an embodiment of the cyclic capture process of acidic gases by solutions of zwitterionic bases in which heat exchangers are used between the sorption tower (scrubbing) and the desorption tower (stripping). This process is particularly suitable for the purification of natural gas. The stream of natural gas **201** containing acidic gases, especially CO₂ or H₂S, comes into contact with the zwitterionic base solution in a sorption tower **202,** under temperature and pressure conditions (T₁ and P₁) typical for sorption, giving rise to the gaseous stream of purified natural gas **203** and a stream of saturated solution of acidic gases **204.** The saturated solution of acidic gases is pumped to the regeneration (or desorption) tower, in which it is subjected to a temperature and pressure condition (T₂ and P₂), which favors the desorption of acidic gases generating a gaseous stream rich in acidic gases **206** and the regenerated zwitterionic base solution. The temperature in the regeneration tower is maintained by reboiler **208,** which heats the regenerated zwitterionic base solution. A part of the regenerated solution **207** that passes through the reboiler is sent back to the sorption tower and another part returns to the desorption tower to maintain the temperature. The regenerated solution stream **207** exchanges heat by heating the solution stream saturated in acidic gases through heat exchanger **209.**

The absorption step of the process promotes the direct or indirect contact, by means of contact membranes, of the gaseous feed stream, with zwitterionic solutions without concentration between 0.5 M and 7 M, temperatures (T₁) between 0 °C to 60 °C and pressures (P₁) between 1 and 150 bar absolute (100 kPa and 15 MPa), typically 40 °C and 1.3 bar to 3 bar absolute (130 kPa to 300 kPa).

The desorption step of the process promotes the regeneration of zwitterionic solutions by heating the solution rich in acidic gases at temperatures (T₂) greater than the absorption temperature (T₁) and/or through the use of pressures (P₂) lower than the absorption pressure (P₁), the desorption temperatures (T₂) being between 60 °C and 150 °C and the desorption pressures between 0 and 10 bar absolute (1 MPa), typically 100 °C and 1 bar absolute (100 kPa).

The process feed gaseous stream can be composed of any gaseous stream or gaseous mixture containing one or more acidic gases, such as carbon dioxide and hydrogen sulfide, in concentrations of 5% to 90%, typically streams of natural gas containing from 1% to 20% CO₂.

The zwitterionic solutions used in the capture process are any of those in which the zwitterionic base is dissolved in water, methanol, ethanol, isopropanol or other hydrocarbon, aliphatic alcohol having from 1 to 18 carbons in the alkyl chain, diols having from 2 to 5 carbon atoms or polyols having from 3 to 6 carbon atoms, ethylene glycol monoalkylethers of the formula H(OCH₂CH₂)ₙOR₂, where n varies from 1 to 4 and R₂ consists of an alkyl group containing from 1 to 12 carbons or ionic liquids functionalized with the hydroxyl group as well as mixing these solvents in appropriate proportions, typically water, ethylene glycol, glycerol and ionic liquids functionalized with the hydroxyl group.

Ionic liquids functionalized with the hydroxyl group are compounds with the general formula A⁺Z⁻, wherein A⁺ represents a quaternary ammonium cation, an imidazolium cation, a pyridinium cation, a pyrrolidinium cation or a phosphonium cation, and Z⁻represents susceptible anions to form a liquid salt with these cations at the absorption temperature.

The cations of ionic liquids include derivatives of the imidazolium cation, the pyridinium cation, the pyrrolidinium cation, the quaternary phosphonium cation or the quaternary ammonium in which, as shown in Figure 25, the R₂₁, R₂₂, R₂₃, R₂₄, R₂₅ and R₂₆ substituents are H, alkylalcohols, alkylether, aryl or alkyl containing between 1 and 10 carbons in each of these groups, typically 1,2-dimethyl-3-(3-hydroxypropyl)-2,3-imidazolium.

The anions of ionic liquids include chloride, bromide, iodide, perchlorate, nitrate, tetrafluoroborate, tetrachloroborate, hexafluorophosphate, trifluoromethanesulfonate, bis(trifluoromethanesulfonyl)imidate, typically NTf₂⁻ and PF₆⁻.

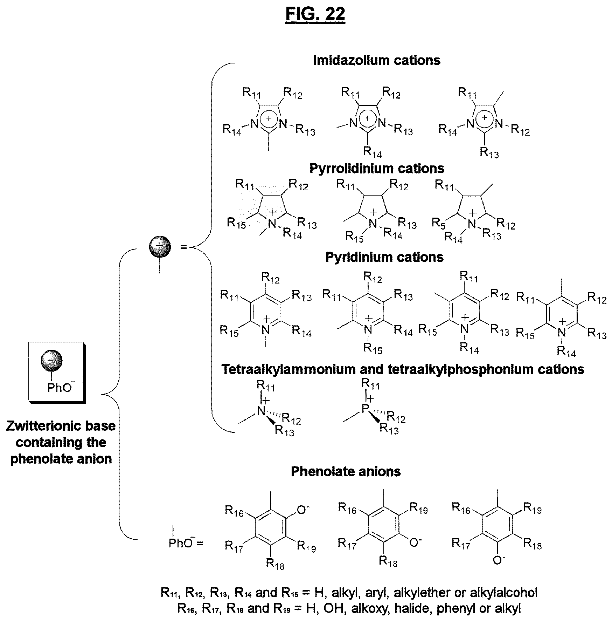

From the mere direct extrapolation of the results disclosed herein, in particular, the results of example 3, example 4, example 5 and example 6 and example 7, it can be concluded that the zwitterionic bases have a predictable effect for capturing acidic gases and that have in their structure an organic cation covalently linked to a phenolate anion; therefore, the process of capturing acidic gases described herein is characterized by the zwitterionic base being an organic compound formed by the covalent association of quaternary ammonium, quaternary phosphonium, and pyridinium cations, pyrrolidinium or imidazolium with substituents, independent of each other, comprising hydrogen and alkyl, aryl, alkylether or alkylalcohol groups containing between 1 and 10 carbons, with the phenolate anion with substituents, independent of each other, comprising hydrogen, halides, phenyl, alkoxy containing between 1 and 10 carbons or alkyl containing between 1 and 10 carbons, according to Figure 22, typically the zwitterionic bases are 1,3-dimethyl-2-(4-oxy-phenyl)-imidazolium and 1,3- dimethyl-2-(3-oxy-phenyl)-imidazolium.

The use of solutions, especially aqueous solutions, of the inner salts called herein zwitterionic bases, object of the present invention, minimizes the main intrinsic inconveniences of large-scale CO₂ capture processes based on aqueous solutions of ethanolamines, as follows:
I) The problems resulting from the loss of mass of the absorbents during the process, such as the increase in costs related to their replacement and the greater operational complexity, are completely solved by the fact that both the absorbent agents and the absorption products are ionic species and, therefore, have negligible vapor pressure;
II) The problems related to thermal and mainly chemical decomposition are minimized by the innovative use of the phenolate functional group in substitution of the amino group, which completely eliminates the decomposition via the formation of carbamates;
III) The costs associated with the energy expenditure of the process are minimized, as estimates suggest that the enthalpy of absorption using aqueous solutions of the inner salts described herein are about 20% lower than the enthalpy of absorption of aqueous solutions of MDEA;
IV) The process embodiment in which ionic liquids are used as solvents also eliminates energy losses resulting from solvent evaporation in the desorption process.

The zwitterion solutions containing the phenolate group, especially aqueous solutions, can be used in gas washing or scrubbing equipment, that is, in conventional equipment intended for the absorption of acidic gases by liquids.

Therefore, in addition to the various technical advantages, this technology is easy to implement, as it allows already-existing CO₂ absorption plants that use aqueous alkanolamine solutions, in particular, in CO₂ and H₂S removal plants from natural gas, are converted into absorption plants by zwitterionic base solutions without all the equipment having to be replaced, requiring only small adaptations, for example, to adjust the energy demand of the process.

### EXAMPLE:

For this work, the following tests were carried out, which represent examples of embodiment of the present invention.

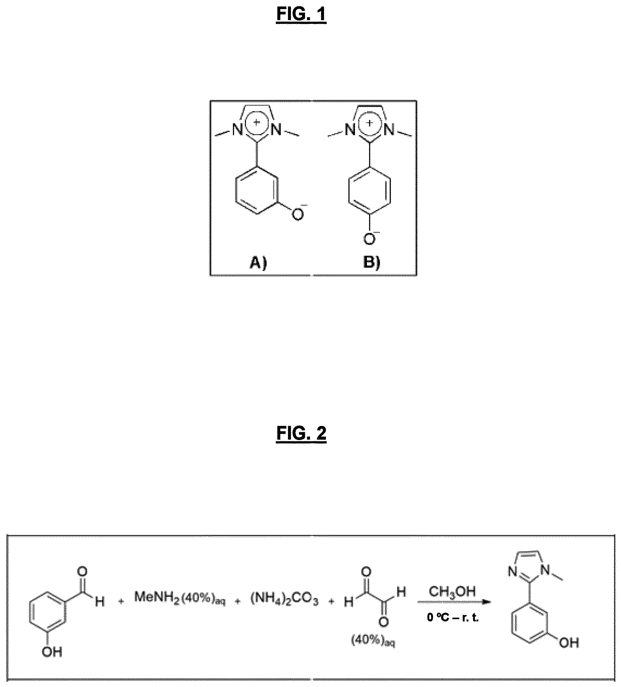

### EXAMPLE 1: Synthesis of 1,3-dimethyl-2-(3-oxy-phenyl)-imidazolium inner salt (Figure 1-A)

The synthesis of 1,3-dimethyl-2-(3-oxy-phenyl)-imidazolium inner salt is carried out in three steps. In the first step, 1-methyl-2(3-hydroxy-phenyl)-imidazole is obtained, as shown in Figure 2.

In a reaction flask, methylamine (40% aqueous solution, 3.3 g; 42 mmol) was added over a solution of 3-hydroxy-benzaldehyde (4.88 g; 40 mmol) in 20 mL of methanol at room temperature. The exothermic reaction causes a slight heating of the reaction mixture and, then, the precipitation of the corresponding imine is observed. The suspension was stirred for another 10 minutes and then ammonium carbonate (2.88 g, 30 mmol) was added. The resulting mixture was cooled to 0 °C using an external ice-water bath. Glyoxal (40% aqueous solution, 5.8 g; 40 mmol) dissolved in 15 mL of CH₃OH was added slowly and under stirring. The reaction mixture was stirred for another 30 minutes at 0 °C, generating a gradual dissolution of the solids. The ice bath was removed and the reaction was stirred for another 24 hours at room temperature, observing the precipitation of the desired reaction product. At the end, 15 mL of water were added to the suspension, the solid was filtered in a Büchner funnel, icy washed with CH₃OH/H₂O 1:1 and dried under reduced pressure. 2.69 g of a slightly yellowish solid were obtained (38.6% yield).

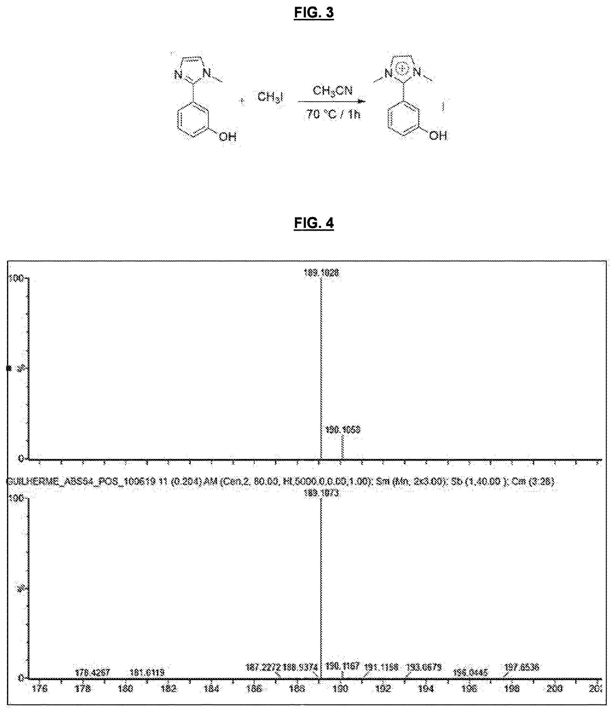

In the second step of the synthesis, 1,3-dimethyl-2(3-hydroxy-phenyl)-imidazolium iodide is obtained, as shown in Figure 3. In a Schlenk flask, 40 mL of acetonitrile, 1-methyl-2-(3-hydroxy-phenyl)-imidazole (6.20 g; 35.6 mmol) and iodomethane (7.60 g; 53.4 mmol). The flask was closed with a rubber septum and the suspension was heated to 70 °C, under stirring with the aid of a magnetic bar, for 1 hour. The solvent in the resulting brownish solution was evaporated to give a solid which was washed with ethyl acetate and then dried under reduced pressure. 10 g of brownish solid were obtained (88.9% yield) according to the ESI(+)-MS spectrum, as illustrated in Figure 4.

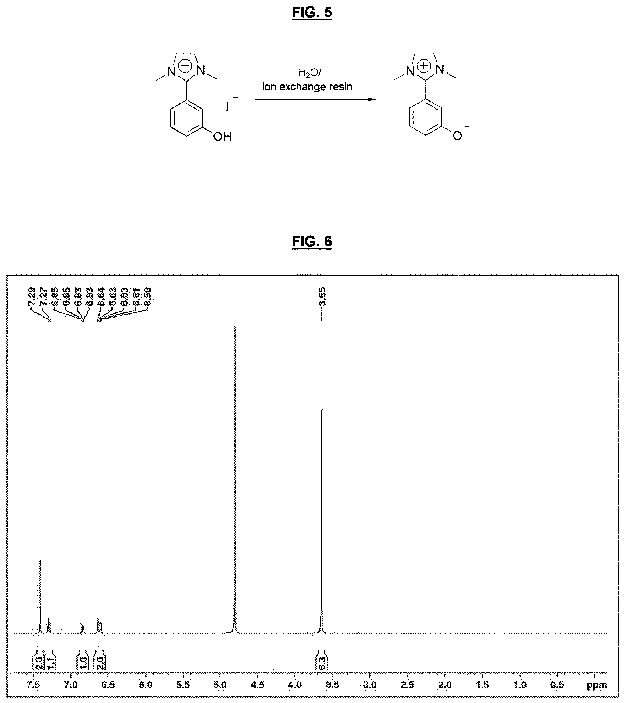

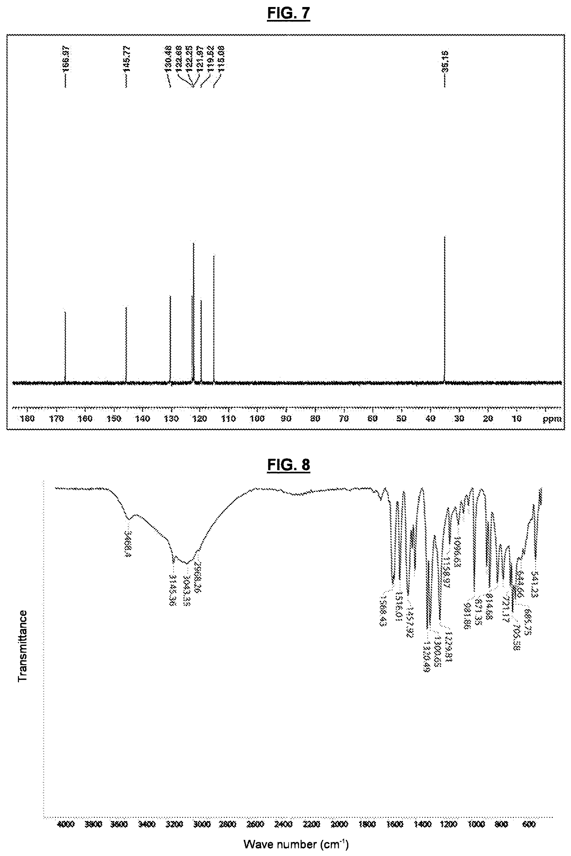

In the third step of the synthesis, the 1,3-dimethyl-2-(3-oxy-phenyl)-imidazolium inner salt is obtained, as shown in Figure 5. In a test tube, there was measured the volume of 14 mL of the ion exchange resin Ambersep 900 OH. The resin was washed three times with distilled water. Next, the resin was added to a solution of 1,3-dimethyl-2-(3-hydroxy-phenyl)-imidazolium iodide (3.16 g, 10.0 mmol) in 10 mL of water. The reaction remained under stirring at room temperature in a beaker for 10 minutes. After this time the ion exchange resin was separated by filtration, and the water in the solution was evaporated under reduced pressure, resulting in 1.8 g of a hygroscopic brown solid (96% yield). The ¹H NMR, ¹³C NMR, infrared spectra are presented, respectively, in Figure 6, Figure 7, and Figure 8.

### EXAMPLE 2: Synthesis of 1,3-dimethyl-2-(4-oxy-phenyl)-imidazolium (Figure 1-B)

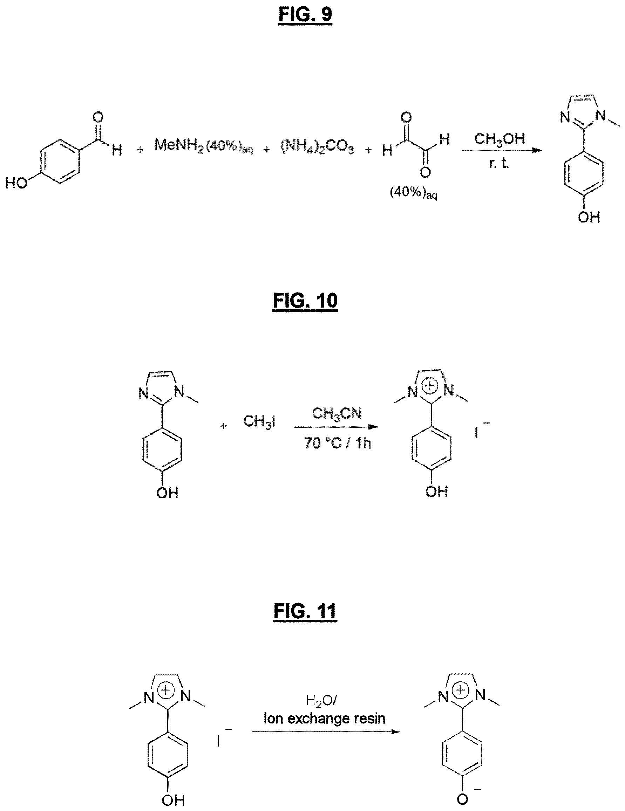

The synthesis of 1,3-dimethyl-2-(4-oxy-phenyl)-imidazolium inner salt is carried out in three steps. In the first step, 1-methyl-2-(4-hydroxy-phenyl)-imidazole is obtained, as shown in Figure 9.

Into a reaction flask, there was added at room temperature methylamine (40% aqueous solution, 3.30 g; 42 mmol) over a solution of 4-hydroxy-benzaldehyde (4.88 g; 40 mmol) in 30 mL of methanol. The reaction mixture warms up a little and is stirred for another 10 minutes. After that time, ammonium carbonate (2.88 g; 30 mmol) was added and to the resulting suspension there was added glyoxal (40% aqueous solution, 5.80 g; 40 mmol) dissolved in 15 mL of CH₃OH, slowly and under stirring. After the addition, the reaction mixture was stirred for another 2 hours at room temperature and then 60 mL of water were added, observing the precipitation of the desired reaction product. The solid was filtered on a Büchner funnel, cold washed with CH₃OH/H₂O 1:1 and dried under reduced pressure. 1.57 g of a slightly yellowish solid were obtained (22.5% yield).

In the second step of the synthesis, 1,3-dimethyl-2-(4-hydroxy-phenyl)-imidazolium iodide was obtained, as shown in Figure 10. In a Schlenk flask, 12 mL of acetonitrile, 1-methyl-2-(3-hydroxy-phenyl)-imidazole (1.04 g; 6.00 mmol) and iodomethane (1.28 g; 9.00 mmol). The flask was closed with a rubber septum and the suspension was heated at 70 °C, under magnetic stirring, for 1 hour. After this period, 5 mL of ethyl acetate was added and the resulting solid was filtered, washed with ethyl acetate and dried under reduced pressure. 1.65 g of brownish crystals were thus obtained (87% yield).

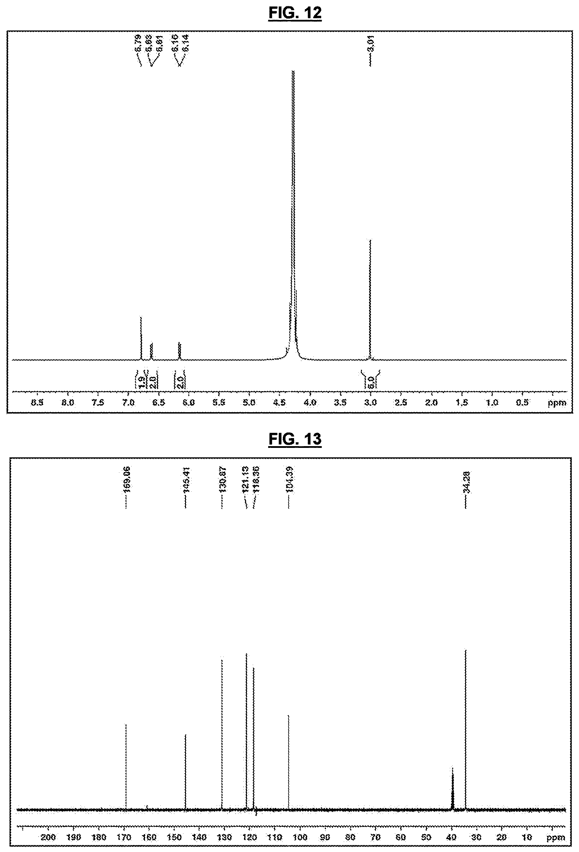

In the third step of the synthesis, the 1,3-dimethyl-2-(4-oxy-phenyl)-imidazolium inner salt was obtained, as shown in Figure 11. In a test tube, there was measured the volume of 14 mL of the ion exchange resin Ambersep 900 OH. The resin was washed three times with distilled water. Then, the resin was added to a solution of 1,3-dimethyl-2-(3-hydroxy-phenyl)-imidazolium iodide (3.16 g; 10 mmol) in 10 mL of water. The reaction remained under stirring at room temperature in a beaker for 10 minutes. After this time, the ion exchange resin was separated by filtration, and the water in the solution was evaporated under reduced pressure, resulting in 1.8 g of a highly hygroscopic brownish solid (96% yield). The ¹H NMR, ¹³C NMR and infrared spectra are presented respectively in Figure 12, Figure 13 and Figure 14.

### Example 3: CO₂ absorption by aqueous solutions of the 1,3-dimethyl-2-(3-oxy-phenyl)-imidazolium and 1,3-dimethyl-2-(4-oxy-phenyl)-imidazolium inner salts at low pressure determined through the pressure drop in the CO₂ reservoir

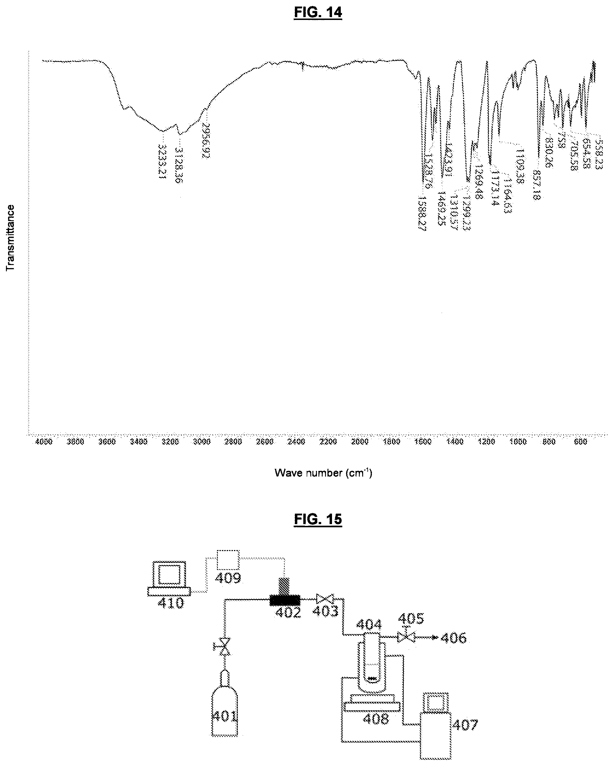

The laboratory tests of absorption and desorption of CO₂ of the solutions of the inner salts were carried out in an experimental apparatus that allows the temperature and pressure of the sorption vessel to be kept constant while the pressure drop in a CO₂ reservoir is monitored for determining the amount of gas absorbed, as shown in Figure 15.

To carry out the test, the reservoir with calibrated volume **402** is previously filled with CO₂ contained in the cylinder **401.** The pressure control valve **403** is adjusted to maintain the pressure of the sorption vessel at 1.3 bar absolute (130 kPa) during the absorption test. The temperature of the sorption vessel **404** is adjusted by circulating, in its jacketed wall, thermal fluid from the thermostatic bath **407** to the absorption temperature (T1), typically 40 °C. Then, 5 mL of 1 M aqueous solution of 1,3-dimethyl-2-(3-oxyphenyl)-imidazolium or 1,3-dimethyl-2-(4-oxy-phenyl)-imidazolium inner salt is added to the reaction vessel. The system was purged with CO₂ for 5 seconds by opening purge valve **406,** and the reservoir **402** was refilled with CO₂ at 4 bar absolute (400 kPa). The absorption process begins when the magnetic stirrer **408** is activated and, from then on, the pressure drop in the CO₂ reservoir is monitored to determine the number of moles of CO₂ consumed. The pressure is monitored through a transducer installed in the CO₂ reservoir coupled to a recorder **409** and a computer **410** for data processing. For the calculations, the ideal gas equation of state and the previously calibrated volume of the reservoir were considered. The end of absorption was considered when the reservoir pressure as a function of time remained at an unchanged level for at least 10 minutes.

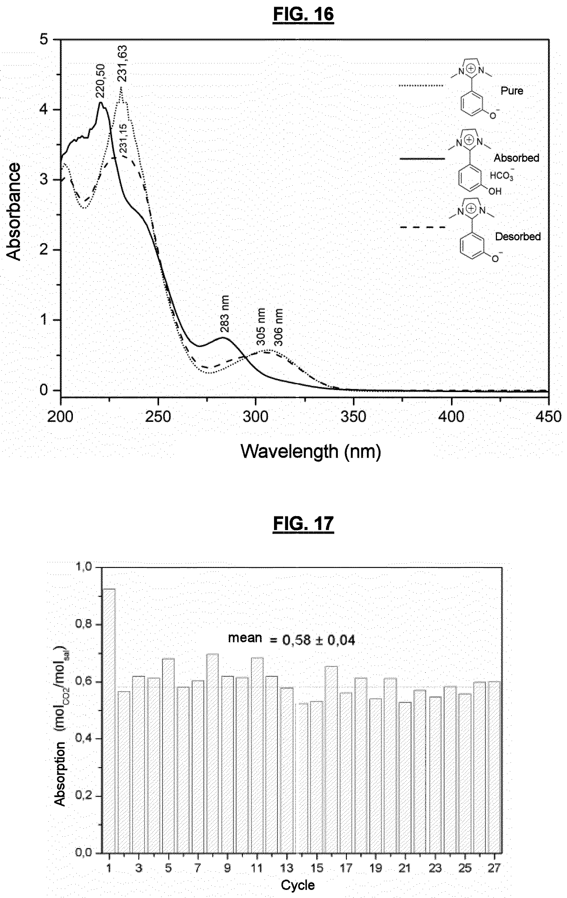

At the end of the absorption step, the sorption vessel **404** was opened and the temperature of the thermostatic bath **407** adjusted to the desorption temperature (T2), typically 100 °C. The system was kept under stirring for 30 minutes to regenerate the zwitterionic solution. With the aim of validating the method and obtaining a parameter for comparison, the absorption test was carried out with the commercial mixture MDEA/piperazine/water normally used in industrial CO₂ absorption processes (Input 1, Table 1). It is important to point out that the tested salts present absorption at wavelengths in the UV-VIS region typical of phenolic decompositions. The wavelengths before and after absorption differ, as shown in Figure 16, suggesting that these systems can be monitored by UV-VIS spectroscopy.

**Table 1. Sorption tests of aqueous solutions of inner salts. ^{a}**

| **Inpu t** | **Absorbent** | **Pressure drop absorption (moles_{CO2}/moles_{sal t}) ^{b}** | **Absorption by ¹³C NMR (moles_{CO2}/moles_{sal t}) ^{c}** | **HCO₃⁻ After desorption (Percentage of desorption) ^{d}** |
|---|---|---|---|---|
| 1 | MDEA/piperazi ne/water(40%/ 10%/ 50% by mass) ^{e} | 0.66 | 0.64 | < 0.3 (> 95%) |
| 2 | 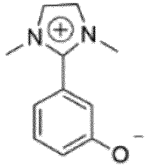 | 0.88 + 0.06 | 0.91 | 0.18 + 0.3 (80%) |
| 3 | 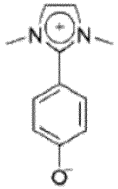 | 0.69 + 0.09 | 0.81 ± 0.05 | < 0.3 (> 95%) |

| | | | | |
|---|---|---|---|---|
| ^{a} Reaction conditions: 5 mL of solution with a concentration of 1 mol/L, temperature of 40 °C and pressure. Mean of at least two analyses ± standard deviation. ^{b} Absorption values expressed in moles CO₂ per moles of absorbent obtained by pressure measurements of 1.3 bar (130 kPa). ^{c} Absorption values expressed in moles CO₂ per moles of absorbent obtained by quantitative ¹³C NMR. ^{d} Desorption percentage = (1-(HCO₃⁻ equivalents after desorption at 100 °C for 30 minutes / ¹³C NMR absorption))*100. ^{e} CO₂ absorption in relation to the number of moles of MDEA + moles of piperazine. | | | | |

### EXAMPLE 4: Recyclability of aqueous solutions of 1,3-dimethyl-2-(3-oxy-phenyl)-imidazolium and 1,3-dimethyl-2-(4-oxy-phenyl)-imidazolium inner salts

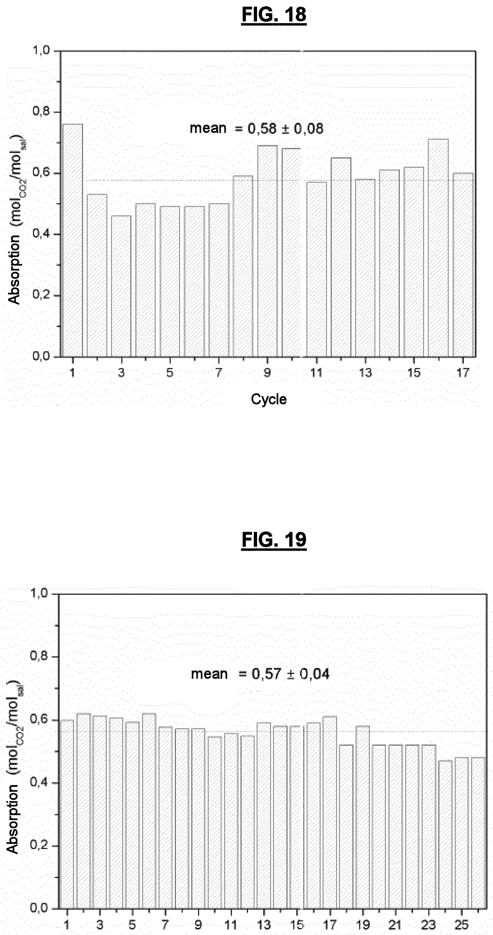

The recyclability of aqueous solutions of the new developed absorbents was tested in cyclic tests of absorption followed by desorption. These tests were carried out in the same experimental apparatus used in Example 1 and shown in Figure 15. The results of these tests are shown in Table 2 and detailed in Figure 17, Figure 18 and Figure 19.

**Table 2. Recyclability tests. ^{a}**

| **Input** | **Absorbent** | **Number of tested cycles** | **Mean Absorption Value ^{b} (moles_{CO2}/molesₛₐₗₜ)** |
|---|---|---|---|
| 1 | MDEA/Piperazine/water (40%/10%/50% by mass) | 26 | 0.57 ± 0.04^{c} |
| 2 | 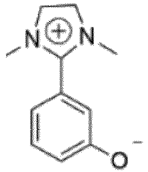 | 27 | 0.58 ± 0.04 |
| 3 | 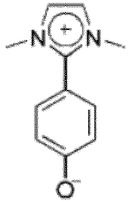 | 17 | 0.58 ± 0.08 |

| | | | |
|---|---|---|---|
| ^{a} Reaction conditions: 5 mL of solution with a concentration of 1 mol/L, absorption temperature 40 °C, and pressure of 1.3 bar (130 kPa), desorption temperature 100 °C, atmospheric pressure (30 min.). ^{b} Absorption values determined through the pressure drop in reservoir and expressed in moles CO₂ per moles of absorbent. Mean represents mean absorption value of the cycles ± standard deviation. ^{c} moles of CO₂ per total moles of amines (MDEA + piperazine) MDEA/piperazine/water40%:10%:50% solution. | | | |

### Example 5: CO₂ absorption by aqueous solutions of the 1,3-dimethyl-2-(3-oxy-phenyl)-imidazolium inner salt determined using the ¹³C nuclear magnetic resonance spectroscopy technique

The capture of CO₂ by zwitterionic solutions of the 1,3-dimethyl-2-(3-oxy-phenyl)-imidazolium inner salt was also studied through quantitative analyses of ¹³C. This analysis methodology was described by SIMON, N. M. et al. "Carbon dioxide capture by aqueous ionic liquid solutions", ChemSusChem, v. 10, p. 4927-4933, 2017 and allows to directly quantify both the chemisorption and the physisorption of CO₂ in the solutions.

To carry out this experiment, a syringe pump coupled to a zirconia NMR tube installed in an NMR spectrometer was used. The syringe pump allows CO₂ pressure control during experiments. The results were analyzed considering the proportion between the integrals of the signals corresponding to the dissolved CO₂ and the bicarbonate formed in relation to the integral of the methyl carbons of the zwitterion. The results are presented in Table 3.

**Table 3. CO₂ absorption determined using ¹³C NMR with pressure variation during spectra acquisition. ^{a}**

| **Absorbent** | **Gauge pressure (bar - × 0.1 MPa)** | **Physisorption (moles_{CO2}/moles_{SALT})** | **Chemisorption (moles_{Co2}/moles_{SALT})** |
|---|---|---|---|
| 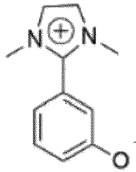 | 10 | 0.07 | 0.99 |
| | 20 | 0.13 | 1.01 |
| | 33 | 0.25 | 1.01 |
| | 40 | 0.27 | 0.99 |

| | | | |
|---|---|---|---|
| ^{a} Reaction conditions: 0.5 mL of solution with a concentration of 2.2 mol/L (1.1 mmol of the inner salt, dissolved in 0.5 mL of D₂O), absorption temperature 40 °C. The NMR spectra were obtained in a Bruker-400-Avance-IIIHD NMR equipment operating at a frequency of 100 MHz for ¹³C equipped with a 5 mm probe with direct detection, with field gradient in z and thermostated via BCU-xtreme with constant temperature and spectral window from -15 ppm to 235 ppm. Used syringe pump: *Xtreme-10 syringpump - deadalus innovation.* | | | |

### EXAMPLE 6: CO₂ absorption by solutions of the 1,3-dimethyl-2-(3-oxy-phenyl)-imidazolium inner salt in protic solvents

One of the main advantages of the CO₂ capture process using zwitterionic solutions is that the zwitterionic bases are not volatile. On the other hand, a fraction of the solvent (usually water) can be lost by evaporation and during the desorption step, which represents energy losses in the process and the need of replacing this solvent. The cyclic CO₂ capture processes with the inner salts disclosed in the present discovery can also be carried out using non-aqueous solvents to minimize, or even eliminate, such mass losses in the desorption step. Alcohols, diols, polyols and ionic liquids functionalized with a hydroxyl group are solvents that, when combined with zwitterionic bases, are capable of reversibly absorbing CO₂ through the formation of the respective alkyl carbonates. In Table 4, some examples of absorption in non-aqueous systems are presented. n-propanol, n-butanol, ethylene glycol or glycerol are alternative solvents with boiling points of 97.0 °C, 117.7 °C, 197.6 °C and 290 °C, respectively. On the other hand, the 1,2-dimethyl-3-(3-hydroxypropyl)-imidazolium bistrifluoromethylsulphonylimidate ionic liquid is a solvent with negligible vapor pressure and represents a totally ionic system for CO₂ capture in which there is certainly no loss of mass by evaporation.

**Table 4. CO₂ absorption through solutions of 1,3-dimethyl-2-(3-oxy-phenyl)-imidazolium inner salt in different protic solvents. ^{a}**

| **Absorbent** | **Solvent** | **Pressure drop absorption (mol_{CO2}/mol_{SALT}) b** | **Absorption by ¹³C NMR (mol_{CO2}/mol_{SALT}) ^{c}** | **Percentage of desorption ^{d}** |
|---|---|---|---|---|
| 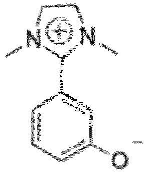 | 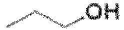 | 0.74 | 0.86 | > 95% |
| | 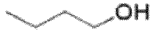 | 0.67 | 0.66 | > 95% |
| | 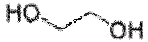 | 0.68 | 0.73 | > 95% |
| | 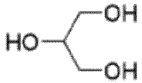 | - | 0.83 | > 95% |
| | 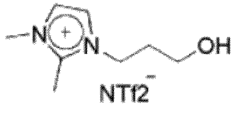 | 0.64 | - | - |

| | | | | |
|---|---|---|---|---|
| ^{a} Reaction conditions: 5 mL of solution with a concentration of 1 mol/L, temperature of 40 °C and pressure. Mean of at least two analyses ± standard deviation. ^{b} Absorption values expressed in moles CO₂ per moles of absorbent obtained by pressure measurements of 1.3 bar (130 kPa). ^{c} Absorption values expressed in moles CO₂ per moles of absorbent obtained by quantitative ¹³C NMR. ^{d} Desorption percentage = (1-(HCO₃⁻ equivalents after desorption at 100 °C for 30 minutes /¹³C NMR absorption))*100. | | | | |

### EXAMPLE 7: Estimation of the enthalpy of absorption in aqueous solutions of the 1,3-dimethyl-2-(3-oxy-phenyl)-imidazolium inner salts

The experimental apparatus shown in Figure 20 was used to determine the global equilibrium constants of the absorption process at temperatures of 10°C, 20 °C, 30 °C, 40 °C, 50 °C and 60 °C. The balance chamber **310** keeps all its components submerged in water with a temperature at the analysis temperature, controlled by the thermostatic bath **309.** The reservoir **302** is filled with CO₂ from the cylinder **301** with the pressure measured by the pressure transducer **305.** The sorption vessel **304** is then evacuated with the aid of a vacuum pump **311** and the zwitterionic-based sorbent solution is injected, through a septum located in the upper part of the sorption vessel, with the aid of a syringe **312.** Then, the sorption vessel is pressurized with CO₂ through the brief opening of the valve **303.** Finally, the pressure drop in the sorption vessel is monitored from the pressure transducer **306,** which sends the data to the recorder **307** and the data is processed in the computer **308.** Enthalpy of absorption of inner salts was determined using the same methodology reported by GABRIELSEN, J. et al. "Model for estimating CO2 solubility in aqueous alkanolamines", Industrial & Engineering Chemistry Research, v. 44, p. 3348-3354, 2005 to estimate the heat of absorption of CO₂ by aqueous solutions of alcoholamines. Table 5 shows the experimental results of the enthalpy of absorption of a commercial MDEA/piperazine/water mixture and for an aqueous solution of the 1,3-dimethyl-2-(3-oxy-phenyl)-imidazolium inner salt.

**Table 5. Estimation of enthalpy of absorption.**

| **Input** | **Absorbent** | **Estimated Enthalpy of Absorption (kJ/mol)** |
|---|---|---|
| 1 | MDEA/Piperazine/water (40%/10%/50% by mass) | -45.0 |
| 2 | 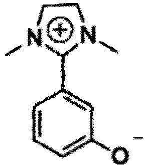 | -36 + 0.8 |

| | | |
|---|---|---|
| ^{a} Reaction conditions: 0.5 mL of solution with a concentration of 1 Mol/L, absorption temperature between 10 °C and 60 °C. Equilibrium pressure between 0.9 bar and 1.4 bar absolute (90 a 140 kPa). | | |

It should be noted that, although the present invention has been described in relation to the attached drawings, these drawing are not intended to have a limiting effect on the scope of the invention, which is defined by the appended claims.

## Claims

1. **A PROCESS OF SYNTHESIS OF ZWITTERIONIC BASES, characterized in that** it comprises three steps:
a) First step: promoting the reaction between a compound of the hydroxy-benzaldehyde type with a primary amine to form the corresponding imine, followed by the cyclization reaction of this imine, by adding an ammonium salt and a vicinal dialdehyde, for obtaining a compound of the 2-(hydroxy-phenyl)-imidazole type, in the presence of a solvent, at temperatures between -10 °C and 80 °C for times between 10 minutes and 24 hours;
b) Second step: promoting the N-alkylation reaction between the compound of 2-(hydroxy-phenyl)-imidazole type obtained in the previous step and an alkylating agent to form a 2-(hydroxy-phenyl)-imidazolium salt, in the presence of solvent, at temperatures between 40 °C and 100 °C and in reaction times between 10 minutes and 24 hours;
c) Third step: promoting the deprotonation of the phenolic OH group of the 2-(hydroxy-phenyl)-imidazolium salt, obtained in the second step, through the reaction of an aqueous solution of this component with a strongly basic ion exchange resin, which results in the formation of the inner or zwitterionic salt of the 2-(oxy-phenyl)-imidazolium type in a time of up to 60 minutes and temperatures between 10 °C and 80 °C.

2. **THE PROCESS** according to claim 1, **characterized in that** the compound of the hydroxy-benzaldehyde type contains at least one hydroxyl group in its aromatic ring and the other substituents of the benzene ring are hydrogen, alkoxy groups containing between 1 and 10 carbons, halides, phenyl or alkyl group or containing between 1 and 10 carbons.

3. **THE PROCESS** according to claim 2, **characterized in that** the compound of the hydroxy-benzaldehyde type is 3-hydroxy-benzaldehyde or 4-hydroxy-benzaldehyde.

4. **THE PROCESS** according to claim 1, **characterized in that** the primary amine has the general formula R1-NH₂ in which the radical R1 comprises an alkyl, aryl, alkylether or alkylalcohol group containing between 1 and 10 carbons.

5. **THE PROCESS** according to claim 4, **characterized in that** the primary amine is methylamine.

6. **THE PROCESS** according to claim 1, **characterized in that** the primary amine is in a proportion of 0.5 to 2 equivalents in relation to the compound of the hydroxy-benzaldehyde type.

7. **THE PROCESS** according to claim 1, **characterized in that** the ammonium salt has the general formula (NH₄)ₙY, where Y comprises the bromide, chloride, sulfate, acetate, carbonate and bicarbonate anions; n has a value of 1 or 2.

8. **THE PROCESS** according to claim 1, **characterized in that** the ammonium salt is in a proportion of 0.3 to 2 equivalents in relation to the compound of the hydroxy-benzaldehyde type.

9. **THE PROCESS** according to claim 1, **characterized in that** the dialdehyde is glyoxal or its analogues with alkyl, aryl, alkylether or alkylalcohol substituents containing 1 to 10 carbons.

10. **THE PROCESS** according to claim 1, **characterized in that** the dialdehyde is in a proportion of 0.5 to 2 equivalents in relation to the compound of the hydroxy-benzaldehyde type.

11. **THE PROCESS** according to claim 1, **characterized in that** the solvent of the first step is water, acetonitrile, ethyl acetate, aliphatic alcohols containing from 1 to 10 carbons in the alkyl chain or a mixture of these in any proportion in the amount of 1 to 100 times the mass of the compound of the hydroxy-benzaldehyde type.

12. **THE PROCESS** according to claim 11, **characterized in that** the solvent is an amount of 10 times the mass of the compound of the hydroxy-benzaldehyde type.

13. **THE PROCESS** according to claim 1, **characterized in that** the alkylating agent has the general formula R9-X, in which R9 comprises an alkyl, arylalkyl, alkylether or alkylalcohol group containing between 1 and 10 carbons, and the X group comprises a halide, alkylsulfonate and alkylsulfate group, in which the alkyl group has between 1 and 5 carbons.

14. **THE PROCESS** according to claim 13, **characterized in that** the alkylating agent is iodomethane or methanesulfonyl chloride.

15. **THE PROCESS** according to claim 1, **characterized in that** the second step occurs at a temperature of 70 °C, under stirring for 1 hour.

16. **THE PROCESS** according to claim 1, **characterized in that** the solvent of the second step is water, acetonitrile, ethyl acetate, aliphatic alcohols containing from 1 to 10 carbons in the alkyl chain or a mixture of these in any proportion in the amount of 1 to 20 times the mass of the compound of the hydroxy-benzaldehyde type.

17. **THE PROCESS** according to claim 16, **characterized in that** the solvent is an amount of 5 times the mass of the compound of the hydroxy-benzaldehyde type.

18. **THE PROCESS** according to claim 1, **characterized in that** the third step occurs at a temperature of 30 °C and under stirring for 10 minutes.

19. **ZWITTERIONIC BASES,** as obtained in the process of claim 1, **characterized in that** they present in their structure an imidazolium cation covalently linked, through carbon C-2, to a phenolate anion, represented by the general formula: 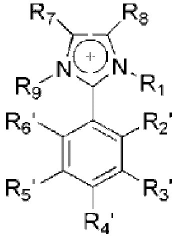 wherein the R₁ and R₉ substituents comprise the alkyl, aryl, alkylether or alkylalcohol group containing 1 to 10 carbons; the R₇ and R₈ substituents of the imidazolium cation comprise hydrogen or alkyl, aryl, alkylether or alkylalcohol groups containing 1 to 10 carbons; and the R₂', R₃', R₄', R₅' and R₆' substituents of the benzene ring are independent of each other and comprise the O⁻ group, hydrogen, hydroxyl, alkoxy groups containing between 1 and 10 carbons, halide, phenyl or alkyl group containing between 1 and 10 carbons, and, necessarily, one of the substituents of the benzene ring is the O⁻ group.

20. **THE ZWITTERIONIC BASES** according to claim 19, **characterized in that** they are solids soluble in water and polar organic solvents, such as alcohols, polyalcohols, acetonitrile and acetone.

21. **A CO₂ CAPTURE PROCESS,** using zwitterionic base as defined in claim 19, **characterized in that** it promotes the contact, under sorption conditions, of a gaseous stream containing acidic gases with a zwitterionic base sorbent solution for the removal of, at least, part of these acidic gases, obtaining a purified gaseous stream and a solution with sorbed acidic gases; and, in parallel, it promotes the regeneration of the sorbent solution, under desorption conditions, to obtain a zwitterionic base solution and a stream of acidic gases.

22. **THE CO₂ CAPTURE PROCESS** according to claim 21, **characterized in that** the gaseous feed stream is composed of a gaseous mixture containing one or more acidic gases, such as carbon dioxide and hydrogen sulfide.

23. A **CO₂ CAPTURE PROCESS** using zwitterionic base as defined in claim 19, **characterized in that** the zwitterionic base is an inner organic salt formed by the covalent association of a quaternary ammonium cation, quaternary phosphonium, pyridinium, pyrrolidinium or imidazolium, with the phenolate anion.

24. **THE CO₂ CAPTURE PROCESS** according to claim 23, **characterized in that** the zwitterionic base containing the quaternary ammonium cation has the formula: 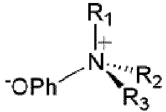 wherein R₁, R₂ and R₃ comprise the alkyl, aryl, alkylether or alkylalcohol groups containing between 1 and 10 carbons; and PhO⁻ represents the phenolate anion with substituents, independent of each other, comprising hydrogen, halides, phenyl, alkoxy containing between 1 and 10 carbons or alkyl containing between 1 and 10 carbons.

25. **THE CO₂ CAPTURE PROCESS** according to claim 23, **characterized in that** the zwitterionic base containing the quaternary phosphonium cation has the formula: 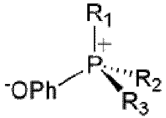 wherein R₁, R₂ and R₃ comprise the alkyl, aryl, alkylether or alkylalcohol groups containing between 1 and 10 carbons; and PhO⁻ represents the phenolate anion with substituents, independent of each other, comprising hydrogen, halides, phenyl, alkoxy containing between 1 and 10 carbons or alkyl containing between 1 and 10 carbons.

26. **THE CO₂ CAPTURE PROCESS** according to claim 23, **characterized in that** the zwitterionic base containing pyridinium has the formula:
wherein R₁, R₂, R₃, R₄ and R₅ comprise hydrogen and alkyl, aryl, alkylether or 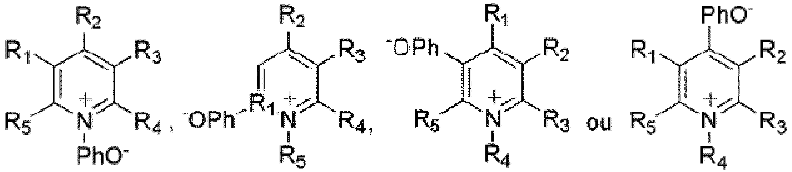 alkylalcohol groups containing between 1 and 10 carbons; and PhO⁻ represents the phenolate anion with substituents, independent of each other, comprising hydrogen, halides, phenyl, alkoxy containing between 1 and 10 carbons or alkyl containing between 1 and 10 carbons.

27. **THE CO₂ CAPTURE PROCESS** according to claim 23, **characterized in that** the zwitterionic base containing the pyrrolidinium cation has the formula: 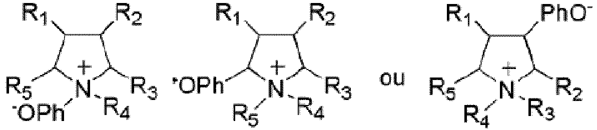 wherein R₁, R₂, R₃, R₄ and R₅ comprise hydrogen and alkyl, aryl, alkylether or alkylalcohol groups containing between 1 and 10 carbons; and PhO⁻ represents the phenolate anion with substituents, independent of each other, comprising hydrogen, halides, phenyl, alkoxy containing between 1 and 10 carbons or alkyl containing between 1 and 10 carbons.

28. **THE CO₂ CAPTURE PROCESS** according to claim 23, **characterized in that** the zwitterionic base containing the imidazolium cation has the formula: 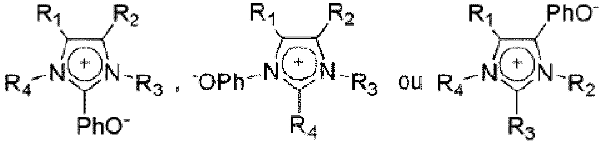 wherein R₁, R₂, R₃ and R₄ comprise hydrogen and alkyl, aryl, alkylether or alkylalcohol groups containing between 1 and 10 carbons; and PhO⁻ represents the phenolate anion with substituents, independent of each other, comprising hydrogen, halides, phenyl, alkoxy containing between 1 and 10 carbons or alkyl containing between 1 and 10 carbons.

29. **THE CO₂ CAPTURE PROCESS** according to claim 21, **characterized in that** the zwitterionic solution is a solution of 1,3-dimethyl-2-(3-oxyphenyl)-imidazolium or 1,3-dimethyl-2-(4-oxy-phenyl)-imidazolium inner salt.

30. **THE CO₂ CAPTURE PROCESS** according to claim 21, **characterized in that** the zwitterionic solution uses as solvent water, methanol, ethanol, isopropanol, aliphatic alcohol having from 1 to 18 carbons in the alkyl chain, diols having from 2 to 5 atoms of carbon or polyols having from 3 to 6 carbon atoms, ethylene glycolmonoalkylethers of the formula H(OCH₂CH₂)ₙOR₂, where n varies from 1 to 4 and R₂ consists of an alkyl group containing from 1 to 12 carbons, ionic liquids functionalized with the hydroxyl group or mixtures thereof.

31. **THE CO₂ CAPTURE PROCESS** according to claim 21, **characterized in that** the absorption condition is: concentration of the zwitterionic base solution between 0.5 M and 5 M, temperature between 0 °C and 60 °C and pressure between 1 and 150 bar absolute (100 kPa and 15 MPa).

32. **THE CO₂ CAPTURE PROCESS** according to claim 21, **characterized in that** the zwitterionic solution of the absorption step presenting a temperature of 40 °C and pressure between 1.3 and 3 bar absolute (130 and 300 kPa).

33. **THE CO₂ CAPTURE PROCESS** according to claim 21, **characterized in that** the desorption condition being: temperature between 60 °C and 150 °C and a pressure between 0 and 10 bar absolute (1 MPa).

34. **THE CO₂ CAPTURE PROCESS** according to claim 33, **characterized in that** the zwitterionic solution of the desorption step presents a temperature of 100 °C and a pressure of 1 absolute bar (100 kPa).

35. **A USE OF THE CO₂ CAPTURE PROCESS,** as defined in claim 21, **characterized in that** it is applied in industrial segments, such as in the energy sector to capture CO₂ from exhaust gases, in the chemical sector to remove CO₂ from process gaseous streams catalysts in which CO₂ can poison the catalysts and especially in the oil and gas sector for the purification of natural gas.

## Patentansprüche

1. PROZESS ZUR SYNTHESE VON ZWITTERIONISCHEN BASEN, **dadurch gekennzeichnet, dass** er drei Schritte umfasst:
a) Erster Schritt: Fördern der Reaktion zwischen einer Verbindung vom Typ Hydroxybenzaldehyd und einem primären Amin, um das entsprechende Imin zu bilden, gefolgt von der Cyclisierungsreaktion dieses Imins durch Hinzufügen eines Ammoniumsalzes und eines vicinalen Dialdehyds, um eine Verbindung vom Typ 2-(Hydroxy-phenyl)-imidazol zu erhalten, in Anwesenheit eines Lösungsmittels, bei Temperaturen zwischen -10 °C und 80 °C für Zeiten zwischen 10 Minuten und 24 Stunden;
b) Zweiter Schritt: Fördern der N-Alkylierungsreaktion zwischen der im vorhergehenden Schritt erhaltenen Verbindung vom Typ 2-(Hydroxy-phenyl)-imidazol und einem Alkylierungsmittel, um ein 2-(Hydroxy-phenyl)-imidazoliumsalz zu bilden, in Anwesenheit eines Lösungsmittels, bei Temperaturen zwischen 40 °C und 100 °C und in Reaktionszeiten zwischen 10 Minuten und 24 Stunden;
c) Dritter Schritt: Fördern der Deprotonierung der phenolischen OH-Gruppe des im zweiten Schritt erhaltenen 2-(Hydroxy-phenyl)-imidazoliumsalzes durch Reaktion einer wässrigen Lösung dieser Komponente mit einem stark basischen Ionenaustauscherharz, was zur Bildung des inneren oder zwitterionischen Salzes vom Typ 2-(Oxy-phenyl)-imidazolium in einer Zeit von bis zu 60 Minuten und bei Temperaturen zwischen 10 °C und 80 °C führt.

2. PROZESS nach Anspruch 1, **dadurch gekennzeichnet, dass** die Verbindung vom Typ Hydroxybenzaldehyd in ihrem aromatischen Ring mindestens eine Hydroxylgruppe enthält und die anderen Substituenten des Benzolrings Wasserstoff, Alkoxygruppen mit 1 bis 10 Kohlenstoffatomen, Halogenide, eine Phenyl- oder Alkylgruppe oder eine Gruppe mit 1 bis 10 Kohlenstoffen sind.

3. PROZESS nach Anspruch 2, **dadurch gekennzeichnet, dass** die Verbindung vom Typ Hydroxybenzaldehyd 3-Hydroxybenzaldehyd oder 4-Hydroxybenzaldehyd ist.

4. PROZESS nach Anspruch 1, **dadurch gekennzeichnet, dass** das primäre Amin die allgemeine Formel R1-NH₂ aufweist, in der der Rest R1 eine Alkyl-, Aryl-, Alkylether- oder Alkylalkoholgruppe mit 1 bis 10 Kohlenstoffen umfasst.

5. PROZESS nach Anspruch 4, **dadurch gekennzeichnet, dass** das primäre Amin Methylamin ist.

6. PROZESS nach Anspruch 1, **dadurch gekennzeichnet, dass** das primäre Amin in einem Verhältnis von 0,5 bis 2 Äquivalenten, bezogen auf die Verbindung vom Typ Hydroxybenzaldehyd, vorliegt.

7. PROZESS nach Anspruch 1, **dadurch gekennzeichnet, dass** das Ammoniumsalz die allgemeine Formel (NH₄)ₙY aufweist, wobei Y die Bromid-, Chlorid-, Sulfat-, Acetat-, Carbonat- und Hydrogencarbonat-Anionen umfasst; n einen Wert von 1 oder 2 aufweist.

8. PROZESS nach Anspruch 1, **dadurch gekennzeichnet, dass** das Ammoniumsalz in einem Verhältnis von 0,3 bis 2 Äquivalenten, bezogen auf die Verbindung vom Typ Hydroxybenzaldehyd, vorliegt.

9. PROZESS nach Anspruch 1, **dadurch gekennzeichnet, dass** das Dialdehyd Glyoxal oder seine Analoga mit Alkyl-, Aryl-, Alkylether- oder Alkylalkoholsubstituenten mit 1 bis 10 Kohlenstoffatomen ist.

10. PROZESS nach Anspruch 1, **dadurch gekennzeichnet, dass** das Dialdehyd in einem Verhältnis von 0,5 bis 2 Äquivalenten, bezogen auf die Verbindung vom Typ Hydroxybenzaldehyd, vorliegt.

11. PROZESS nach Anspruch 1, **dadurch gekennzeichnet, dass** das Lösungsmittel des ersten Schritts Wasser, Acetonitril, Ethylacetat, aliphatische Alkohole mit 1 bis 10 Kohlenstoffatomen in der Alkylkette oder ein Gemisch aus diesen in einem beliebigen Verhältnis in der 1- bis 100-fachen Menge der Masse der Verbindung vom Typ Hydroxybenzaldehyd ist.

12. PROZESS nach Anspruch 11, **dadurch gekennzeichnet, dass** die Menge des Lösungsmittels das 10-fache der Masse der Verbindung vom Typ Hydroxybenzaldehyd ist.

13. PROZESS nach Anspruch 1, **dadurch gekennzeichnet, dass** das Alkylierungsmittel die allgemeine Formel R9-X aufweist, in der R9 eine Alkyl-, Arylalkyl-, Alkylether- oder Alkylalkoholgruppe mit 1 bis 10 Kohlenstoffen umfasst und die X-Gruppe eine Halogenid-, Alkylsulfonat- und Alkylsulfatgruppe umfasst, in der die Alkylgruppe 1 bis 5 Kohlenstoffe aufweist.

14. PROZESS nach Anspruch 13, **dadurch gekennzeichnet, dass** das Alkylierungsmittel lodmethan oder Methansulfonylchlorid ist.

15. PROZESS nach Anspruch 1, **dadurch gekennzeichnet, dass** der zweite Schritt bei einer Temperatur von 70 °C und unter Rühren für 1 Stunde stattfindet.

16. PROZESS nach Anspruch 1, **dadurch gekennzeichnet, dass** das Lösungsmittel des zweiten Schritts Wasser, Acetonitril, Ethylacetat, aliphatische Alkohole mit 1 bis 10 Kohlenstoffatomen in der Alkylkette oder ein Gemisch aus diesen in einem beliebigen Verhältnis in der 1- bis 20-fachen Menge der Masse der Verbindung vom Typ Hydroxybenzaldehyd ist.

17. PROZESS nach Anspruch 16, **dadurch gekennzeichnet, dass** die Menge des Lösungsmittels das 5-fache der Masse der Verbindung vom Typ Hydroxybenzaldehyd ist.

18. PROZESS nach Anspruch 1, **dadurch gekennzeichnet, dass** der dritte Schritt bei einer Temperatur von 30 °C und unter Rühren für 10 Minuten stattfindet.

19. ZWITTERIONISCHE BASEN, wie sie im Prozess nach Anspruch 1 erhalten werden, **dadurch gekennzeichnet, dass** sie in ihrer Struktur ein Imidazoliumkation aufweisen, das über den Kohlenstoff C-2 kovalent an ein Phenolatanion gebunden ist, dargestellt durch die allgemeine Formel: 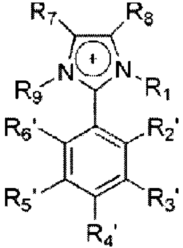 wobei die R₁- und R₉-Substituenten die Alkyl-, Aryl-, Alkylether- oder Alkylalkoholgruppe mit 1 bis 10 Kohlenstoffen umfassen; die R₇- und R₈-Substituenten des Imidazoliumkations Wasserstoff oder Alkyl-, Aryl-, Alkylether- oder Alkylalkoholgruppen mit 1 bis 10 Kohlenstoffen umfassen und die R₂'-, R₃'-, R₄'-, R₅'- und R₆'-Substituenten des Benzolrings unabhängig voneinander sind und die O⁻-Gruppe, Wasserstoff, Hydroxyl-, Alkoxygruppen mit 1 bis 10 Kohlenstoffatomen, Halogenid-, Phenyl- oder Alkylgruppe mit 1 bis 10 Kohlenstoffatomen umfassen, wobei einer der Substituenten des Benzolrings notwendigerweise die O⁻-Gruppe ist.

20. ZWITTERIONISCHE BASEN nach Anspruch 19, **dadurch gekennzeichnet, dass** sie in Wasser und polaren organischen Lösungsmitteln wie Alkoholen, Polyalkoholen, Acetonitril und Aceton lösliche Feststoffe sind.

21. PROZESS ZUR CO₂-ABSCHEIDUNG unter Verwendung einer zwitterionischen Base nach Anspruch 19, **dadurch gekennzeichnet, dass** er den Kontakt eines Gasstroms, der saure Gase enthält, mit einer Sorbenslösung einer zwitterionischen Base unter Sorptionsbedingungen fördert, um mindestens einen Teil dieser sauren Gase zu entfernen, wobei ein gereinigter Gasstrom und eine Lösung mit sorbierten sauren Gasen erhalten wird; und dass er parallel dazu die Regeneration der Sorbenslösung unter Desorptionsbedingungen fördert, um eine Lösung einer zwitterionischen Base und einen Strom von sauren Gasen zu erhalten.

22. PROZESS ZUR CO₂-ABSCHEIDUNG nach Anspruch 21, **dadurch gekennzeichnet, dass** der gasförmige Zuführungsstrom aus einem gasförmigen Gemisch besteht, das ein oder mehrere saure Gase, wie Kohlendioxid und Wasserstoffsulfid, enthält.

23. PROZESS ZUR CO₂-ABSCHEIDUNG unter Verwendung einer zwitterionischen Base nach Anspruch 19, **dadurch gekennzeichnet, dass** die zwitterionische Base ein inneres organisches Salz ist, das durch die kovalente Verknüpfung eines quaternären Ammoniumkations, quaternären Phosphoniums, Pyridiniums, Pyrrolidiniums oder Imidazoliums mit dem Phenolatanion gebildet wird.

24. PROZESS ZUR CO₂-ABSCHEIDUNG nach Anspruch 23, **dadurch gekennzeichnet, dass** die zwitterionische Base, die das quaternäre Ammoniumkation enthält, folgende Formel aufweist: 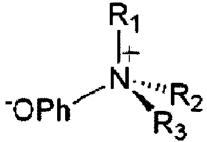 wobei R₁, R₂ und R₃ die Alkyl-, Aryl-, Alkylether- oder Alkylalkoholgruppen mit 1 bis 10 Kohlenstoffen umfassen; und PhO⁻ das Phenolatanion mit Substituenten darstellt, die unabhängig voneinander Wasserstoff, Halogenide, Phenyl, Alkoxy mit 1 bis 10 Kohlenstoffen oder Alkyl mit 1 bis 10 Kohlenstoffen umfassen.

25. PROZESS ZUR CO₂-ABSCHEIDUNG nach Anspruch 23, **dadurch gekennzeichnet, dass** die zwitterionische Base, die das quaternäre Phosphoniumkation enthält, folgende Formel aufweist: 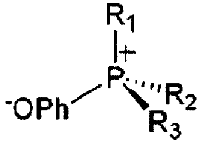 wobei R₁, R₂ und R₃ die Alkyl-, Aryl-, Alkylether- oder Alkylalkoholgruppen mit 1 bis 10 Kohlenstoffen umfassen; und PhO⁻ das Phenolatanion mit Substituenten darstellt, die unabhängig voneinander Wasserstoff, Halogenide, Phenyl, Alkoxy mit 1 bis 10 Kohlenstoffen oder Alkyl mit 1 bis 10 Kohlenstoffen umfassen.

26. PROZESS ZUR CO₂-ABSCHEIDUNG nach Anspruch 23, **dadurch gekennzeichnet, dass** die zwitterionische Base, die Pyridinium enthält, folgende Formel aufweist: 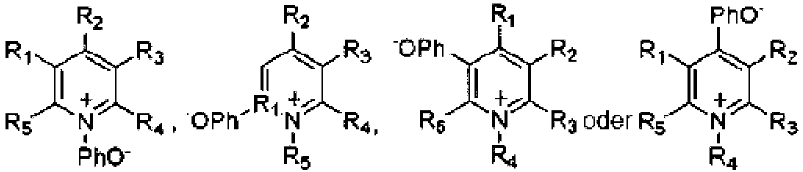 wobei R₁, R₂, R₃, R₄ und R₅ Wasserstoff und Alkyl-, Aryl-, Alkylether- oder Alkylalkoholgruppen mit 1 bis 10 Kohlenstoffen umfassen; und PhO⁻ das Phenolatanion mit Substituenten darstellt, die unabhängig voneinander Wasserstoff, Halogenide, Phenyl, Alkoxy mit 1 bis 10 Kohlenstoffen oder Alkyl mit 1 bis 10 Kohlenstoffen umfassen.

27. PROZESS ZUR CO₂-ABSCHEIDUNG nach Anspruch 23, **dadurch gekennzeichnet, dass** die zwitterionische Base, die das Pyrrolidiniumkation enthält, folgende Formel aufweist: 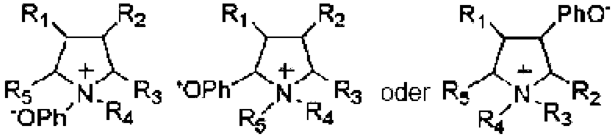 wobei R₁, R₂, R₃, R₄ und R₅ Wasserstoff und Alkyl-, Aryl-, Alkylether- oder Alkylalkoholgruppen mit 1 bis 10 Kohlenstoffen umfassen; und PhO⁻ das Phenolatanion mit Substituenten darstellt, die unabhängig voneinander Wasserstoff, Halogenide, Phenyl, Alkoxy mit 1 bis 10 Kohlenstoffen oder Alkyl mit 1 bis 10 Kohlenstoffen umfassen.

28. PROZESS ZUR CO₂-ABSCHEIDUNG nach Anspruch 23, **dadurch gekennzeichnet, dass** die zwitterionische Base, die das Imidazoliumkation enthält, folgende Formel aufweist: 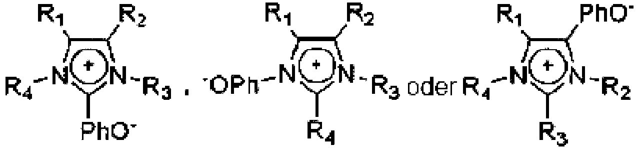 wobei R₁, R₂, R₃, und R₄ Wasserstoff und Alkyl-, Aryl-, Alkylether- oder Alkylalkoholgruppen mit 1 bis 10 Kohlenstoffen umfassen; und PhO⁻ das Phenolatanion mit Substituenten darstellt, die unabhängig voneinander Wasserstoff, Halogenide, Phenyl, Alkoxy mit 1 bis 10 Kohlenstoffen oder Alkyl mit 1 bis 10 Kohlenstoffen umfassen.

29. PROZESS ZUR CO₂-ABSCHEIDUNG nach Anspruch 21, **dadurch gekennzeichnet, dass** die zwitterionische Lösung eine Lösung des inneren Salzes von 1,3-Dimethyl-2-(3-oxyphenyl)-imidazolium oder 1,3-Dimethyl-2-(4-oxy-phenyl)-imidazolium ist.

30. PROZESS ZUR CO₂-ABSCHEIDUNG nach Anspruch 21, **dadurch gekennzeichnet, dass** die zwitterionische Lösung als Lösungsmittel Wasser, Methanol, Ethanol, Isopropanol, aliphatische Alkohole mit 1 bis 18 C-Atomen in der Alkylkette, Diole mit 2 bis 5 C-Atomen oder Polyole mit 3 bis 6 C-Atomen, Ethylenglykolmonoalkylether der Formel H(OCH₂CH₂)ₙOR₂, wobei n im Bereich von 1 bis 4 liegt und R₂ aus einer Alkylgruppe mit 1 bis 12 C-Atomen besteht, mit der Hydroxylgruppe funktionalisierte ionische Flüssigkeiten oder Gemische davon verwendet.

31. PROZESS ZUR CO₂-ABSCHEIDUNG nach Anspruch 21, **dadurch gekennzeichnet, dass** die Absorptionsbedingung folgende ist: Konzentration der Lösung der zwitterionischen Base zwischen 0,5 M und 5 M, Temperatur zwischen 0 °C und 60 °C und Druck zwischen 1 und 150 bar absolut (100 kPa und 15 MPa).

32. PROZESS ZUR CO₂-ABSCHEIDUNG nach Anspruch 21, **dadurch gekennzeichnet, dass** die zwitterionische Lösung des Absorptionsschritts eine Temperatur von 40 °C und einen Druck zwischen 1,3 und 3 bar absolut (130 und 300 kPa) aufweist.

33. PROZESS ZUR CO₂-ABSCHEIDUNG nach Anspruch 21, **dadurch gekennzeichnet, dass** die Desorptionsbedingung Folgende ist: Temperatur zwischen 60 °C und 150 °C und ein Druck zwischen 0 und 10 bar absolut (1 MPa).

34. PROZESS ZUR CO₂-ABSCHEIDUNG nach Anspruch 33, **dadurch gekennzeichnet, dass** die zwitterionische Lösung des Desorptionsschritts eine Temperatur von 100 °C und einen Druck von 1 bar absolut (100 kPa) aufweist.

35. VERWENDUNG DES PROZESSES ZUR CO₂-ABSCHEIDUNG nach Anspruch 21, **dadurch gekennzeichnet, dass** er in industriellen Segmenten eingesetzt wird, wie im Energiesektor, um CO₂ aus Abgasen abzuscheiden, im Chemiesektor, um CO₂ aus Katalysatoren von Prozessgasströmen zu entfernen, in denen CO₂ die Katalysatoren vergiften kann, und insbesondere im Öl- und Gassektor zur Reinigung von Erdgas.

## Revendications

1. Procédé de synthèse de bases zwitterioniques, **caractérisé en ce qu'**il comprend trois étapes :
a) première étape : promotion de la réaction entre un composé du type hydroxybenzaldéhyde et une amine primaire pour former l'imine correspondante, suivie de la réaction de cyclisation de ladite imine par addition d'un sel d'ammonium et d'un dialdéhyde vicinal, afin d'obtenir un composé du type 2-(hydroxy-phényl)-imidazole, en présence d'un solvant, à des températures comprises entre -10 °C et 80 °C pendant des durées comprises entre 10 minutes et 24 heures ;
b) deuxième étape : promotion de la réaction de N-alkylation entre le composé du type 2-(hydroxy-phényl)-imidazole obtenu à l'étape précédente et un agent alkylant pour former un sel d'imidazolium du type 2-(hydroxy-phényl)-imidazolium, en présence d'un solvant, à des températures comprises entre 40 °C et 100 °C et pendant des durées de réaction comprises entre 10 minutes et 24 heures ;
c) troisième étape : promotion de la déprotonation du groupe OH phénolique du sel du type 2-(hydroxy-phényl)-imidazolium obtenu à la deuxième étape, par réaction d'une solution aqueuse de ce composé avec une résine échangeuse d'ions fortement basique, ce qui conduit à la formation du sel interne ou zwitterionique du type 2-(oxy-phényl)-imidazolium, en un temps allant jusqu'à 60 minutes et à des températures comprises entre 10 °C et 80 °C.

2. Procédé selon la revendication 1, **caractérisé en ce que** le composé du type hydroxy-benzaldéhyde contient au moins un groupe hydroxyle dans son cycle aromatique, et **en ce que** les autres substituants du cycle benzénique sont de l'hydrogène, des groupes alkoxy comportant de 1 à 10 atomes de carbone, des halogénures, un groupe phényl ou un groupe alkyle comportant de 1 à 10 atomes de carbone.

3. Procédé selon la revendication 2, **caracterisé en ce que** le composé du type hydroxybenzaldéhyde est le 3-hydroxybenzaldéhyde ou le 4-hydroxybenzaldéhyde.

4. Procédé selon la revendication 1, **caractérisé en ce que** l'amine primaire présente la formule générale R1-NH2, dans laquelle le radical R1 comprend un groupe alkyle, aryle, alkyléther ou alkylalcool comportant de 1 à 10 atomes de carbone.

5. Procédé selon la revendication 4, **caractérisé en ce que** l'amine primaire est la méthylamine.

6. Procédé selon la revendication 1, **caractérisé en ce que** l'amine primaire est utilisée dans une proportion de 0,5 à 2 équivalents par rapport au composé du type hydroxybenzaldéhyde.

7. Procédé selon la revendication 1, **caractérisé en ce que** le sel d'ammonium présente la formule générale (NH₄)ₙY, dans laquelle Y comprend les anions bromure, chlorure, sulfate, acétate, carbonate et bicarbonate, et n a une valeur de 1 ou 2.

8. Procédé selon la revendication 1, **caractérisé en ce que** le sel d'ammonium est utilisé dans une proportion de 0,3 à 2 équivalents par rapport au composé du type hydroxybenzaldéhyde.

9. Procédé selon la revendication 1, **caractérisé en ce que** le dialdéhyde est le glyoxal ou ses analogues comportant des substituants alkyle, aryle, alkyléther ou alkylalcool contenant de 1 à 10 atomes de carbone.

10. Procédé selon la revendication 1, **caractérisé en ce que** le dialdéhyde est utilisé dans une proportion de 0,5 à 2 équivalents par rapport au composé du type hydroxybenzaldéhyde.

11. Procédé selon la revendication 1, **caractérisé en ce que** le solvant de la première étape est l'eau, l'acétonitrile, l'acétate d'éthyle, un alcool aliphatique comportant de 1 à 10 atomes de carbone dans la chaîne alkyle, ou un mélange de ceux-ci en toute proportion, dans une quantité comprise entre 1 et 100 fois la masse du composé du type hydroxybenzaldéhyde.

12. Procédé selon la revendication 11, **caractérisé en ce que** le solvant est utilisé dans une quantité égale à 10 fois la masse du composé du type hydroxybenzaldéhyde.

13. Procédé selon la revendication 1, **caractérisé en ce que** l'agent alkylant présente la formule générale R9-X, dans laquelle R9 comprend un groupe alkyle, arylalkyle, alkyléther ou alkylalcool comportant de 1 à 10 atomes de carbone, et le groupe X comprend un groupe halogénure, alkylsulfonate ou alkylsulfate, dans lequel le groupe alkyle comporte de 1 à 5 atomes de carbone.

14. Procédé selon la revendication 13, **caractérisé en ce que** l'agent alkylant est l'iodométhane ou le chlorure de méthanesulfonyle.

15. Procédé selon la revendication 1, **caractérisé en ce que** la deuxième étape s'effectue à une température de 70 °C, sous agitation pendant 1 heure.

16. Procédé selon la revendication 1, **caractérisé en ce que** le solvant de la deuxième étape est l'eau, l'acétonitrile, l'acétate d'éthyle, un alcool aliphatique comportant de 1 à 10 atomes de carbone dans la chaîne alkyle, ou un mélange de ceux-ci en toute proportion, dans une quantité comprise entre 1 et 20 fois la masse du composé du type hydroxybenzaldéhyde.

17. Procédé selon la revendication 16, **caractérisé en ce que** le solvant est utilisé dans une quantité égale à 5 fois la masse du composé du type hydroxybenzaldéhyde.

18. Procédé selon la revendication 1, **caractérisé en ce que** la troisième étape s'effectue à une température de 30 °C, sous agitation pendant 10 minutes.

19. Bases zwitterioniques, obtenues selon le procédé de la revendication 1, **caractérisées en ce qu'**elles présentent dans leur structure un cation imidazolium lié de manière covalente, par le carbone C-2, à un anion phénolate, représentées par la formule générale : 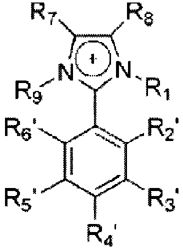 dans laquelle les substituants R₁ et R₉ comprennent un groupe alkyle, aryle, alkyléther ou alkylalcool comportant de 1 à 10 atomes de carbone ; les substituants R₇ et R₈ du cation imidazolium comprennent de l'hydrogène ou des groupes alkyle, aryle, alkyléther ou alkylalcool comportant de 1 à 10 atomes de carbone ; et les substituants R₂', R₃', R₄', R₅' et R₆' du cycle benzénique sont indépendants les uns des autres et comprennent le groupe O⁻, de l'hydrogène, un groupe hydroxyle, des groupes alkoxy comportant de 1 à 10 atomes de carbone, un halogénure, un groupe phényl ou un groupe alkyle comportant de 1 à 10 atomes de carbone, et, nécessairement, l'un des substituants du cycle benzénique est le groupe O⁻.

20. Bases zwitterioniques selon la revendication 19, **caractérisées en ce qu'**elles sont solides et solubles dans l'eau et dans des solvants organiques polaires, tels que les alcools, les polyalcools, l'acétonitrile et l'acétone.

21. Procédé de capture du CO2 utilisant une base zwitterionique telle que définie dans la revendication 19, **caractérisé en ce qu'**il promeut le contact, dans des conditions d'adsorption, d'un flux gazeux contenant des gaz acides avec une solution adsorbante de base zwitterionique afin d'éliminer au moins une partie de ces gaz acides, permettant d'obtenir un flux gazeux purifié et une solution contenant les gaz acides adsorbés ; et, parallèlement, il promeut la régénération de la solution adsorbante, dans des conditions de désorption, afin d'obtenir une solution de base zwitterionique et un flux de gaz acides.

22. Procédé de capture du CO₂ selon la revendication 21, **caractérisé en ce que** le flux gazeux d'alimentation est constitué d'un mélange gazeux contenant un ou plusieurs gaz acides, tels que le dioxyde de carbone et le sulfure d'hydrogène.

23. Procédé de capture du CO₂ utilisant une base zwitterionique telle que définie dans la revendication 19, **caractérisé en ce que** la base zwitterionique est un sel organique interne formé par l'association covalente d'un cation ammonium quaternaire, phosphonium quaternaire, pyridinium, pyrrolidinium ou imidazolium avec l'anion phénolate.

24. Procédé de capture du CO₂ selon la revendication 23, **caractérisé en ce que** la base zwitterionique contenant le cation ammonium quaternaire présente la formule : 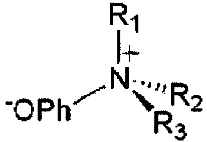 dans laquelle R₁, R₂ et R₃ comprennent des groupes alkyle, aryle, alkyléther ou alkylalcool comportant de 1 à 10 atomes de carbone, et PhO⁻ représente l'anion phénolate comportant des substituants indépendants les uns des autres comprenant de l'hydrogène, des halogénures, un groupe phényl, un groupe alkoxy comportant de 1 à 10 atomes de carbone ou un groupe alkyle comportant de 1 à 10 atomes de carbone.

25. Procédé de capture du CO₂ selon la revendication 23, **caractérisé en ce que** la base zwitterionique contenant le cation phosphonium quaternaire présente la formule : 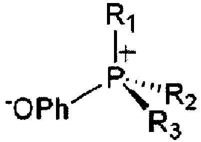 dans laquelle R₁, R₂ et R₃ comprennent des groupes alkyle, aryle, alkyléther ou alkylalcool comportant de 1 à 10 atomes de carbone, et PhO⁻ représente l'anion phénolate comportant des substituants indépendants les uns des autres comprenant de l'hydrogène, des halogénures, un groupe phényl, un groupe alkoxy comportant de 1 à 10 atomes de carbone ou un groupe alkyle comportant de 1 à 10 atomes de carbone.

26. Procédé de capture du CO₂ selon la revendication 23, **caractérisé en ce que** la base zwitterionique contenant le cation phosphonium quaternaire présente la formule :
dans laquelle R₁, R₂ R₃, R₄ et R₅ comprennent des groupes alkyle, aryle, alkyléther ou 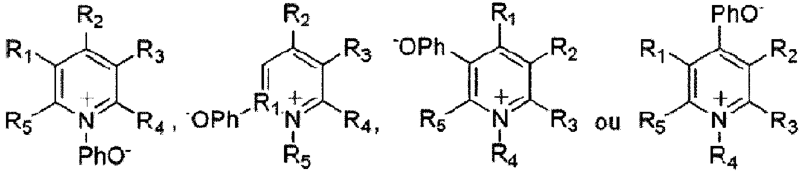 alkylalcool comportant de 1 à 10 atomes de carbone, et PhO⁻ représente l'anion phénolate comportant des substituants indépendants les uns des autres comprenant de l'hydrogène, des halogénures, un groupe phényl, un groupe alkoxy comportant de 1 à 10 atomes de carbone ou un groupe alkyle comportant de 1 à 10 atomes de carbone.

27. Procédé de capture du CO₂ selon la revendication 23, **caractérisé en ce que** la base zwitterionique contenant le cation pyrrolidinium présente la formule : 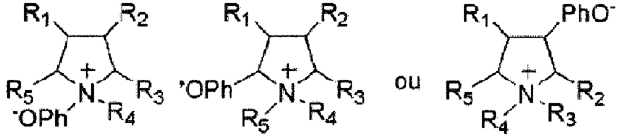 dans laquelle R₁, R₂ R₃, R₄ et R₅ comprennent des groupes hydrogène et alkyle, aryle, alkyléther ou alkylalcool comportant de 1 à 10 atomes de carbone, et PhO⁻représente l'anion phénolate comportant des substituants indépendants les uns des autres comprenant de l'hydrogène, des halogénures, un groupe phényl, un groupe alkoxy comportant de 1 à 10 atomes de carbone ou un groupe alkyle comportant de 1 à 10 atomes de carbone.

28. Procédé de capture du CO₂ selon la revendication 23, **caractérisé en ce que** la base zwitterionique contenant le cation imidazolium présente la formule : 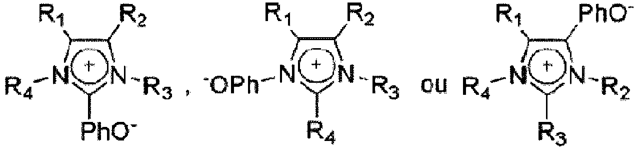 dans laquelle R₁, R₂ R₃, R₄ et R₅ comprennent des groupes hydrogène et alkyle, aryle, alkyléther ou alkylalcool comportant de 1 à 10 atomes de carbone, et PhO⁻représente l'anion phénolate comportant des substituants indépendants les uns des autres comprenant de l'hydrogène, des halogénures, un groupe phényl, un groupe alkoxy comportant de 1 à 10 atomes de carbone ou un groupe alkyle comportant de 1 à 10 atomes de carbone.

29. Procédé de capture du CO₂ selon la revendication 21, **caractérisé en ce que** la solution zwitterionique est une solution de sel interne de 1,3-diméthyl-2-(3-oxyphényl)-imidazolium ou de 1,3-diméthyl-2-(4-oxyphényl)-imidazolium.

30. Procédé de capture du CO₂ selon la revendication 21, **caractérisé en ce que** la solution zwitterionique utilise comme solvant l'eau, le méthanol, l'éthanol, l'isopropanol, un alcool aliphatique comportant de 1 à 18 atomes de carbone dans la chaîne alkyle, un diol comportant de 2 à 5 atomes de carbone ou un polyol comportant de 3 à 6 atomes de carbone, des éthers monoalkyliques de l'éthylèneglycol de formule H(OCH₂CH₂)ₙOR₂, où n varie de 1 à 4 et R₂ consiste en un groupe alkyle contenant de 1 à 12 atomes de carbone, des liquides ioniques fonctionnalisés par un groupe hydroxyle, ou des mélanges de ceux-ci.

31. Procédé de capture du CO₂ selon la revendication 21, **caractérisé en ce que** la condition d'absorption est la suivante : concentration de la solution de base zwitterionique comprise entre 0,5 M et 5 M, température comprise entre 0 °C et 60 °C et pression comprise entre 1 et 150 bar absolus (100 kPa et 15 MPa).

32. Procédé de capture du CO₂ selon la revendication 21, **caractérisé en ce que** la solution zwitterionique de l'étape d'absorption présente une température de 40 °C et une pression comprise entre 1,3 et 3 bar absolus (130 et 300 kPa).

33. Procédé de capture du CO₂ selon la revendication 21, **caractérisé en ce que** la condition de désorption est la suivante : température comprise entre 60 °C et 150 °C et pression comprise entre 0 et 10 bar absolus (1 MPa).

34. Procédé de capture du CO₂ selon la revendication 33, **caractérisé en ce que** la solution zwitterionique de l'étape de désorption présente une température de 100 °C et une pression de 1 bar absolu (100 kPa).

35. Utilisation du procédé de capture du CO₂ tel que défini dans la revendication 21, **caractérisée en ce qu'**il est appliqué dans des secteurs industriels tels que le secteur de l'énergie pour la capture du CO₂ à partir des gaz d'échappement, dans le secteur chimique pour l'élimination du CO₂ des flux gazeux de procédé dans lesquels le CO₂ peut empoisonner les catalyseurs, et en particulier dans le secteur pétrolier et gazier pour la purification du gaz naturel.
